(19) Europäisches Patentamt
European Patent Office
Office européen des brevets

(11) EP 1 339 700 B1

(12) EUROPEAN PATENT SPECIFICATION

(45) Date of publication and mention of the grant of the patent:
**21.11.2007 Bulletin 2007/47**

(21) Application number: **01983478.7**

(22) Date of filing: **11.09.2001**

(51) Int Cl.:
***C07D 249/20*** *(2006.01)*

(86) International application number:
**PCT/EP2001/010478**

(87) International publication number:
**WO 2002/024668 (28.03.2002 Gazette 2002/12)**

(54) **PROCESS FOR THE PREPARATION OF BENZOTRIAZOLES**

VERFAHREN ZUR HERSTELLUNG VON BENZTRIAZOLEN

PROCEDE DE PREPARATION DE BENZOTRIAZOLES

(84) Designated Contracting States:
**AT BE CH CY DE DK ES FI FR GB GR IE IT LI LU MC NL PT SE TR**

(30) Priority: **20.09.2000 CH 183000**

(43) Date of publication of application:
**03.09.2003 Bulletin 2003/36**

(73) Proprietor: **Ciba Specialty Chemicals Holding Inc.**
**4057 Basel (CH)**

(72) Inventors:
- **FISCHER, Walter**
  **CH-4153 Reinach (CH)**
- **FRITZSCHE, Katharina**
  **79576 Weil am Rhein (DE)**
- **WOLF, Walter**
  **79713 Bad Säckingen (DE)**
- **BORE, Lothar**
  **79618 Rheinfelden (DE)**

(56) References cited:
**US-A- 2 904 544**

- **HARTMANN ET AL: JOURNAL FUER PRAKTISCHE CHEMIE, LEIPZIG, DE, vol. 332, no. 3, 1990, pages 394-402, XP001027061 ISSN: 0021-8383**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a process for the preparation of benzotriazoles, which are suitable for the stabilisation of organic materials against light-induced degradation.

The best methods used hitherto for the preparation of benzotriazoles are all based on processes in which it is necessary for a reducing agent to be used at least once. Such processes are described, for example, in U.S. 5 276 161 and U.S. 5 977 219. The reduction step produces intensely coloured, undesired secondary products, which have to be removed from the desired benzotriazoles, for example by chromatography, at great expense.

U.S. 2,904,544 discloses the preparation of new nitrogen-containing compounds having two benzenoid rings, to each of which are attached in the ortho position two nitrogens which in turn are bonded only to nitrogen. An intermediate in the preparation of these compounds is 2-(o-azidophenyl)benzotriazole.

H. Hartmann et al., "Journal für praktische Chemie, Band 332, Heft 3", 1990, pages 331 - 335, discloses a simple route to 2H-naphtho[1,2-c]1,2,3-triazoles. 1-Arylazo-2-chloro-naphthalenes and 2,4-diarylazo-1-chloronaphthalenes are transformed by the reaction with sodium azide into 2-arylsubstituted 2H-naphtho[1,2-c]1,2,3-triazoles which exhibit fluorescence if they are not nitro or arylazo substituted. If the educts bear different bounded halogens only the naphthalene linked chlorine is substituted by the azide ion.

There is accordingly still a need to find an efficient process for the preparation of benzotriazoles that yields the benzotriazoles, for example, without the use of reducing agents, and accordingly does not have the above-mentioned disadvantages.

The present invention accordingly relates to a process for the preparation of compounds of formula I

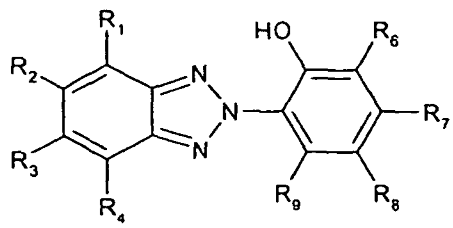

(I)

wherein

$R_1$, $R_2$. $R_3$, $R_4$, $R_6$, $R_7$, $R_8$ and $R_9$ are each independently of the others hydrogen, halogen, $-SO_3H$, $-SO_3^- M_a^+$, hydroxy, carboxy, cyano, nitro, $C_1$-$C_{25}$alkyl, $C_1$-$C_{25}$alkyl substituted by halogen, hydroxy, carboxy, cyano, $C_1$-$C_{18}$alkoxycarbonyl, $C_1$-$C_4$alkoxy or by amino; $C_2$-$C_{24}$alkenyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$cycloalkyl; $C_1$-$C_{18}$alkoxy, $C_1$-$C_{18}$alkylthio, $C_1$-$C_{18}$alkylsulfonyl, unsubstituted or $C_1$-$C_4$alkyl-substituted phenylsulfonyl, unsubstituted or $C_1$-$C_4$alkyl-substituted phenylthio; amino, $C_1$-$C_4$alkylamino, di($C_1$-$C_4$alkyl)amino, $C_1$-$C_{25}$alkanoyl, $C_1$-$C_{25}$alkoxycarbonyl, $C_1$-$C_{25}$alkanoyloxy, $C_1$-$C_{25}$alkanoylamino, $C_3$-$C_{25}$alkyl interrupted by oxygen, sulfur or by

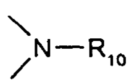 ;

$C_3$-$C_{25}$alkoxy interrupted by oxygen, sulfur or by

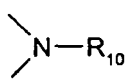 ;

$C_3$-$C_{25}$alkanoyloxy interrupted by oxygen, sulfur or by

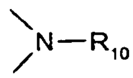

$C_3$-$C_{25}$alkoxycarbonyl interrupted by oxygen, sulfur or by

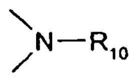

$C_6$-$C_9$cycloalkoxycarbonyl, $C_6$-$C_9$cycloalkylcarbonyloxy, unsubstituted or $C_1$-$C_{12}$alkyl-substituted benzoyloxy; $-(CH_2)_p-COR_{11}$ or $-(CH_2)_qOH$, or, further, the radicals $R_6$ and $R_7$ or the radicals $R_7$ and $R_8$ or the radicals $R_8$ and $R_9$, together with the carbon atoms to which they are bonded, form a benzo ring, $R_3$ in addition is a radical of formula II

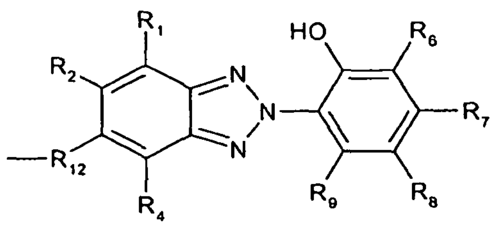

(II),

and $R_6$ in addition is a radical of formula III

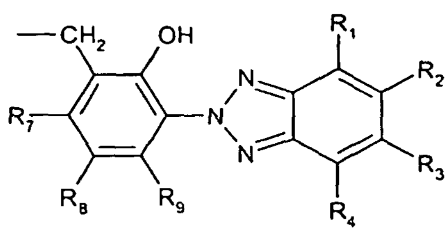

(III),

$R_{10}$ is hydrogen or $C_1$-$C_8$alkyl,
$R_{11}$ is hydroxy,

$$\left[-O^{-}\ \frac{1}{r}M_b^{r+}\right] ,$$

$C_1$-$C_{18}$alkoxy,

$$-O\left[CH_2CH_2-O\right]_m-H ,$$

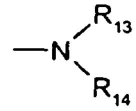

or a radical of formula IV

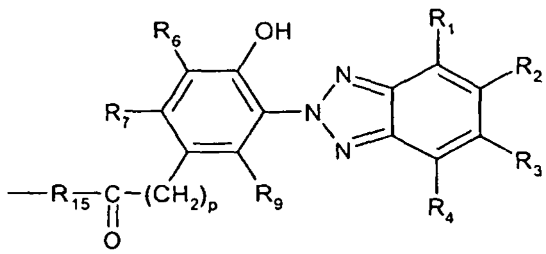

(IV),

$R_{12}$ is -SO-, $-SO_2-$, $-SO-R_{16}-SO-$, $-SO_2-R_{16}-SO_2-$,

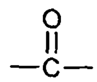 , 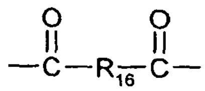 ,

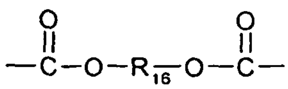 , 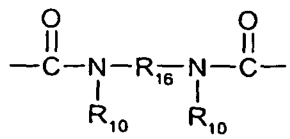 or 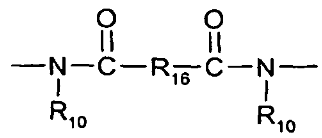 ,

$R_{13}$ and $R_{14}$ are each independently of the other hydrogen or $C_1$-$C_{18}$alkyl,
$R_{15}$ is

$$—O—R_{17}—O— ,$$

$R_{16}$ is $C_2$-$C_{12}$alkylene, $C_5$-$C_{12}$cycloalkylene, or $C_8$-$C_{12}$alkylene interrupted or terminated by cyclohexylene;
$R_{17}$ is $C_2$-$C_{12}$alkylene, or $C_4$-$C_{12}$alkylene interrupted by oxygen, sulfur or by

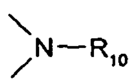 ,

$M_a$ is a monovalent metal cation,
$M_b$ is an r-valent metal cation,
m is an integer from 1 to 20,
p is 0, 1 or 2,
q is 1, 2, 3, 4, 5 or 6, and
r is 1, 2 or 3,

which process comprises reacting a compound of formula V

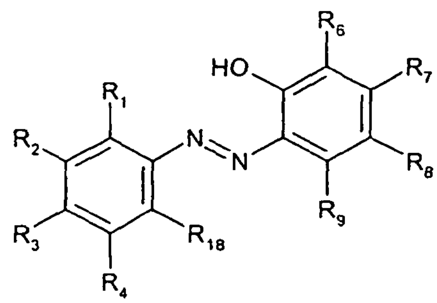

(V)

wherein

$R_1$, $R_2$, $R_3$, $R_4$, $R_6$, $R_7$, $R_8$ and $R_9$ are as defined hereinbefore, $R_3$ in addition being a radical of formula VI

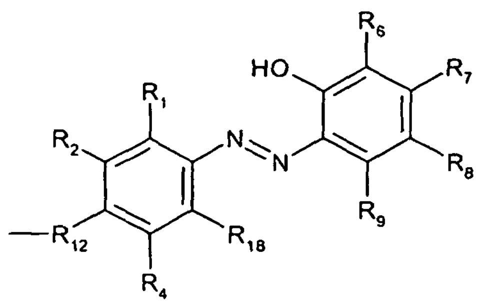

(VI),

$R_6$ in addition being a radical of formula VII

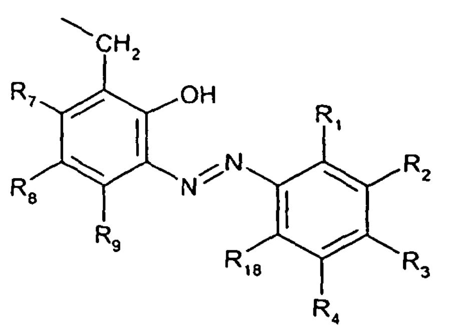

(VII)

and $R_{11}$ in addition being a radical of formula VIII

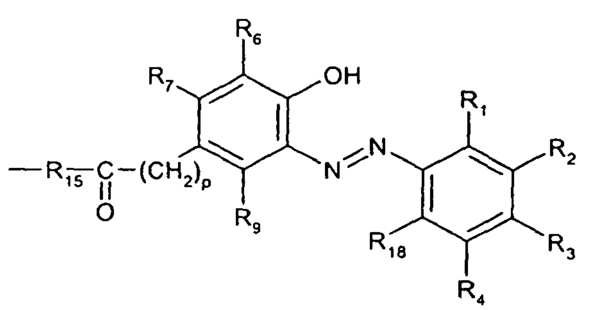

(VIII),

$R_{18}$ is halogen, nitro,

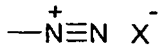, 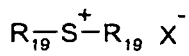,

$-SO_3H$,

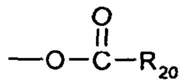,

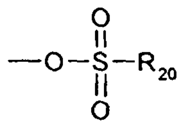

or $C_1$-$C_{18}$alkoxy,

$R_{19}$ is $C_1$-$C_{18}$alkyl, unsubstituted or $C_1$-$C_4$alkyl-substituted phenyl; or unsubstituted or $C_1$-$C_4$alkyl-substituted $C_5$-$C_8$cycloalkyl,

$R_{20}$ is $C_1$-$C_{18}$alkyl, unsubstituted or $C_1$-$C_4$alkyl-, halo- or nitro-substituted phenyl; unsubstituted or $C_1$-$C_4$alkyl-substituted $C_5$-$C_8$cycloalkyl; or fluorine-substituted $C_1$-$C_{18}$alkyl, and

$X^-$ is chloride, bromide, iodide, hydroxide, nitrate or nitrite,

with an azide compound of formula IX

$$M^{n+} (N_3^-)_n \qquad (IX)$$

wherein

M is an n-valent metal cation,

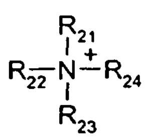

or $P^+(R_{25})_4$,
$R_{21}$, $R_{22}$, $R_{23}$ and $R_{24}$ are each independently of the others hydrogen or $C_1$-$C_{18}$alkyl, $R_{25}$ is $C_1$-$C_{18}$alkyl, and nis1,2or3.

Halogen is, for example, fluorine, chlorine, bromine or iodine. Chlorine is preferred.

Alkyl having up to 25 carbon atoms is a branched or unbranched radical, for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl, dodecyl, 1,1,3,3,5,5-hexamethylhexyl, tridecyl, tetradecyl, pentadecyl, hexadecyl, heptadecyl, octadecyl, eicosyl or docosyl. One of the preferred meanings of $R_1$, $R_2$, $R_3$, $R_4$, $R_5$, $R_7$ and $R_9$ is, for example, $C_1$-$C_{18}$alkyl, especially $C_1$-$C_{12}$alkyl, e.g. $C_1$-$C_4$alkyl. An especially preferred meaning of $R_6$ is $C_1$-$C_{12}$alkyl, especially $C_1$-$C_8$alkyl, e.g. $C_1$-$C_5$alkyl. An especially preferred meaning of $R_8$ is $C_1$-$C_{12}$alkyl, especially $C_1$-$C_{10}$alkyl, e.g. $C_1$-$C_8$alkyl. A preferred meaning of $R_{10}$ is $C_1$-$C_8$alkyl, especially

$C_1$-$C_6$alkyl, e.g. $C_1$-$C_4$alkyl. One of the preferred meanings of $R_{13}$ and $R_{14}$ is $C_1$-$C_{12}$alkyl, especially $C_1$-$C_8$alkyl, e.g. methyl or ethyl. A preferred meaning of $R_{20}$ is $C_1$-$C_{12}$alkyl, especially $C_1$-$C_8$alkyl, e.g. $C_1$-$C_4$alkyl. One of the preferred meanings of $R_{21}$, $R_{22}$, $R_{23}$ and $R_{24}$ is $C_1$-$C_{12}$alkyl, especially $C_1$-$C_4$alkyl, e.g. n-butyl.

$C_1$-$C_{25}$Alkyl substituted by halogen, hydroxy, carboxy, cyano, $C_1$-$C_{18}$alkoxycarbonyl, $C_1$-$C_4$alkoxy or by amino is a branched or unbranched radical, for example trifluoromethyl, hydroxymethyl, carboxymethyl, cyanomethyl, methoxycarbonylmethyl, ethoxycarbonylmethyl, aminomethyl, pentafluoroethyl, 1-hydroxyethyl, 2-hydroxyethyl, 1-carboxyethyl, 2-carboxyethyl, 1-cyanoethyl, 2-cyanoethyl, 1-methoxycarbonylethyl, 2-methoxycarbonylethyl, 1-ethoxycarbonylethyl, 2-ethoxycarbonylethyl, 2-methoxyethyl, 1-aminoethyl, 2-aminoethyl, 3-chloropropyl, 1-hydroxypropyl, 2-hydroxypropyl, 3-hydroxypropyl, 1-carboxypropyl, 2-carboxypropyl, 3-carboxypropyl, 1-cyanopropyl, 2-cyanopropyl, 3-cyanopropyl, 1-methoxycarbonylpropyl, 2-methoxycarbonylpropyl, 3-methoxycarbonylpropyl, 2-methoxypropyl, 3-methoxypropyl, 1-aminopropyl, 2-aminopropyl or 3-aminopropyl.

Alkenyl having from 2 to 24 carbon atoms is a branched or unbranched radical, for example vinyl, propenyl, 2-butenyl, 3-butenyl, isobutenyl, n-2,4-pentadienyl, 3-methyl-2-butenyl, n-2-octenyl, n-2-dodecenyl, isododecenyl, oleyl, n-2-octadecenyl or n-4-octadecenyl. Preference is given to alkenyl having from 3 to 18, especially from 3 to 12, for example from 2 to 6, carbon atoms.

$C_7$-$C_9$Phenylalkyl is, for example, benzyl, α-methylbenzyl, α,α-dimethylbenzyl or 2-phenylethyl. A preferred meaning of $R_6$ and $R_8$ is, for example, α,α-dimethylbenzyl.

Unsubstituted or $C_1$-$C_4$alkyl-, halo- or nitro-substituted phenyl that contains preferably from 1 to 3 substituents, especially 1 or 2 substituents, is, for example, o-, m- or p-methylphenyl, p-chlorophenyl, p-nitrophenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2-methyl-6-ethylphenyl, 4-tert-butylphenyl, 2-ethylphenyl, 2,6-diethylphenyl, 2,4-dichlorophenyl, 2,4,6-trichlorophenyl, 2,4-dinitrophenyl or 2,6-dichloro-4-nitrophenyl.

Unsubstituted or $C_1$-$C_4$alkyl-substituted $C_5$-$C_8$cycloalkyl is, for example, cyclopentyl, methylcyclopentyl, dimethylcyclopentyl, cyclohexyl, methylcyclohexyl, dimethylcyclohexyl, trimethylcyclohexyl, tert-butylcyclohexyl, cycloheptyl or cyclooctyl. Preference is given to cyclohexyl.

Alkoxy having up to 18 carbon atoms is a branched or unbranched radical, for example methoxy, ethoxy, propoxy, isopropoxy, n-butoxy, isobutoxy, pentyloxy, isopentyloxy, hexyloxy, heptyloxy, octyloxy, decyloxy, tetradecyloxy, hexadecyloxy or octadecyloxy. Preference is given to alkoxy having from 1 to 12, especially from 1 to 8, e.g. from 1 to 6, carbon atoms. An especially preferred meaning of $R_2$, $R_{11}$ and $R_{18}$ is methoxy.

Alkylthio having up to 25 carbon atoms is a branched or unbranched radical, for example methylthio, ethylthio, propylthio, isopropylthio, n-butylthio, isobutylthio, pentylthio, isopentylthio, hexylthio, heptylthio, octylthio, decylthio, tetradecylthio, hexadecylthio or octadecylthio. Preference is given to alkylthio having from 1 to 12, especially from 1 to 8, e.g. from 1 to 6, carbon atoms.

Alkylsulfonyl having up to 18 carbon atoms is a branched or unbranched radical, for example methylsulfonyl, ethylsulfonyl, n-propylsulfonyl, isopropylsulfonyl, n-butylsulfonyl, isobutylsulfonyl, tert-butylsulfonyl, 2-ethylbutylsulfonyl, n-pentylsulfonyl, isopentylsulfonyl, 1-methylpentylsulfonyl, 1,3-dimethylbutylsulfonyl, n-hexylsulfonyl, 1-methylhexylsulfonyl, n-heptylsulfonyl, isoheptylsulfonyl, 1,1,3,3-tetramethyfbutylsulfonyl, 1-methylheptylsulfonyl, 3-methylheptylsulfonyl, n-octylsulfonyl, 2-ethylhexylsulfonyl, 1,1,3-trimethylhexylsulfonyl, 1,1,3,3-tetramethylpentylsulfonyl, nonylsulfonyl, decylsulfonyl, undecylsulfonyl, 1-methylundecylsulfonyl, dodecylsulfonyl, 1,1,3,3,5,5-hexamethylhexylsulfonyl, tridecylsulfonyl, tetradecylsulfonyl, pentadecylsulfonyl, hexadecylsulfonyl, heptadecylsulfonyl or octadecylsulfonyl. A preferred meaning of $R_2$ and $R_3$ is $C_1$-$C_{12}$alkylsulfonyl, especially $C_1$-$C_8$alkylsulfonyl, e.g. $C_1$-$C_4$alkylsulfonyl.

Unsubstituted or $C_1$-$C_4$alkyl-substituted phenylsulfonyl that contains preferably from 1 to 3 alkyl groups, especially 1 or 2 alkyl groups, is, for example, o-, m- or p-methylphenylsulfonyl, 2,3-dimethylphenylsulfonyl, 2,4-dimethylphenylsulfonyl, 2,5-dimethylphenylsulfonyl, 2,6-dimethylphenylsulfonyl, 3,4-dimethylphenylsulfonyl, 3,5-dimethylphenylsulfonyl, 2-methyl-6-ethylphenylsulfonyl, 4-tert-butylphenylsulfonyl, 2-ethylphenylsulfonyl or 2,6-diethylphenylsulfonyl.

Unsubstituted or $C_1$-$C_4$alkyl-substituted phenylthio that contains preferably from 1 to 3 alkyl groups, especially 1 or 2 alkyl groups, is, for example, o-, m- or p-methylphenylthio, 2,3-dimethylphenylthio, 2,4-dimethylphenylthio, 2,5-dimethylphenylthio, 2,6-dimethylphenylthio, 3,4-dimethylphenylthio, 3,5-dimethylphenylthio, 2-methyl-6-ethylphenylthio, 4-tert-butylphenylthio, 2-ethylphenylthio or 2,6-diethylphenylthio.

Alkylamino having up to 4 carbon atoms is a branched or unbranched radical, for example methylamino, ethylamino, propylamino, isopropylamino, n-butylamino, isobutylamino or tert-butylamino.

Di($C_1$-$C_4$alkyl)amino denotes that the two radicals are each independently of the other branched or unbranched, for example dimethylamino, methylethylamino, diethylamino, methyl-n-propylamino, methylisopropylamino, methyl-n-butylamino, methylisobutylamino, ethylisopropylamino, ethyl-n-butylamino, ethylisobutylamino, ethyl-tert-butylamino, diethylamino, diisopropylamino, isopropyl-n-butylamino, isopropylisobutylamino, di-n-butylamino or di-isobutylamino.

Alkanoyl having up to 25 carbon atoms is a branched or unbranched radical, for example formyl, acetyl, propionyl, butanoyl, pentanoyl, hexanoyl, heptanoyl, octanoyl, nonanoyl, decanoyl, undecanoyl, dodecanoyl, tridecanoyl,

tetradecanoyl, pentadecanoyl, hexadecanoyl, heptadecanoyl, octadecanoyl, eicosanoyl or docosanoyl. Alkanoyl has preferably from 2 to 18, especially from 2 to 12, e.g. from 2 to 6, carbon atoms. Special preference is given to acetyl.

Alkoxycarbonyl having up to 25 carbon atoms is a branched or unbranched radical, for example methoxycarbonyl, ethoxycarbonyl, propoxycarbonyl, isopropoxycarbonyl, n-butoxycarbonyl, isobutoxycarbonyl, pentyloxycarbonyl, isopentyloxycarbonyl, hexyloxycarbonyl, heptyloxycarbonyl, octyloxycarbonyl, decyloxycarbonyl, tetradecyloxycarbonyl, hexadecyloxycarbonyl or octadecyloxycarbonyl. Preference is given to alkoxycarbonyl having from 1 to 18, especially from 1 to 12, e.g. from 1 to 8, carbon atoms. An especially preferred meaning is methoxycarbonyl or ethoxycarbonyl.

Alkanoyloxy having up to 25 carbon atoms is a branched or unbranched radical, for example formyloxy, acetoxy, propionyloxy, butanoyloxy, pentanoyloxy, hexanoyloxy, heptanoyloxy, octanoyloxy, nonanoyloxy, decanoyloxy, undecanoyloxy, dodecanoyloxy, tridecanoyloxy, tetradecanoyloxy, pentadecanoyloxy, hexadecanoyloxy, heptadecanoyloxy, octadecanoyloxy, eicosanoyloxy or docosanoyloxy. Preference is given to alkanoyloxy having from 2 to 18, especially from 2 to 12, e.g. from 2 to 6, carbon atoms. Special preference is given to acetoxy.

Alkanoylamino having up to 25 carbon atoms is a branched or unbranched radical, for example formylamino, acetylamino, propionylamino, butanoylamino, pentanoylamino, hexanoylamino, heptanoylamino, octanoylamino, nonanoylamino, decanoylamino, undecanoylamino, dodecanoylamino, tridecanoylamino, tetradecanoylamino, pentadecanoylamino, hexadecanoylamino, heptadecanoylamino, octadecanoylamino, eicosanoylamino or docosanoylamino. Preference is given to alkanoylamino having from 2 to 18, especially from 2 to 12, e.g. from 2 to 6, carbon atoms.

$C_3$-$C_{25}$Alkyl interrupted by oxygen, sulfur or by

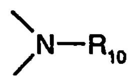

is, for example, $CH_3$-O-$CH_2CH_2$-, $CH_3$-S-$CH_2CH_2$-, $CH_3$-N($CH_3$)-$CH_2$-, $CH_3$-O-$CH_2CH_2$-O-$CH_2CH_2$-, $CH_3$-(O-$CH_2CH_2$-)$_2$O-$CH_2CH_2$-, $CH_3$-(O-$CH_2CH_2$-)$_3$O-$CH_2CH_2$- or $CH_3$-(O-$CH_2CH_2$-)$_4$0-$CH_2CH_2$-.

$C_3$-$C_{25}$Alkoxy interrupted by oxygen, sulfur or by

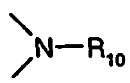

is, for example, $CH_3$-O-$CH_2CH_2O$-, $CH_3$-S-$CH_2CH_2O$-, $CH_3$-N($CH_3$)-$CH_2CH_2O$-, $CH_3$-O-$CH_2CH_2$-O-$CH_2CH_2O$-, $CH_3$-(O-$CH_2CH_2$-)$_2$O-$CH_2CH_2O$-, $CH_3$-(O-$CH_2CH_2$-)$_3$O-$CH_2CH_2O$- or $CH_3$-(O-$CH_2CH_2$-)$_4$O-$CH_2CH_2O$-.

$C_3$-$C_{25}$Akanoyloxy interrupted by oxygen, sulfur or by

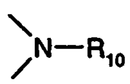

is, for example, $CH_3$-O-$CH_2COO$-, $CH_3$-S-$CH_2COO$-, $CH_3$-N($CH_3$)-$CH_2COO$-, $CH_3$-O-$CH_2CH_2$-O-$CH_2COO$-, $CH_3$-(O-$CH_2CH_{2.}$)$_2$O-$CH_2COO$-, $CH_3$-(O-$CH_2CH_2$-)$_3$O-$CH_2COO$- or $CH_3$-(O-$CH_2CH_2$-)$_4$O-$CH_2COO$-.

$C_3$-$C_{25}$Alkoxycarbonyl interrupted by oxygen, sulfur or by

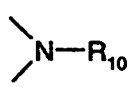

is, for example, $CH_3$-O-$CH_2CH_2OCO$-, $CH_3$-S-$CH_2CH_2OCO$-, $CH_3$-N($CH_3$)-$CH_2CH_2OCO$-, $CH_3$-O-$CH_2CH_2$-O-$CH_2CH_2OCO$-, $CH_3$-(O-$CH_2CH_2$-)$_2$O-$CH_2CH_2OCO$-, $CH_3$-(O-$CH_2CH_2$-)$_3$O-$CH_2CH_2OCO$- or $CH_3$-(O-$CH_2CH_2$-)$_4$O-$CH_2CH_2OCO$-.

$C_6$-$C_9$Cycloalkoxycarbonyl is, for example, cyclohexyloxycarbonyl, cycloheptyloxycarbonyl, cyclooctyloxycarbonyl or cyclononyloxycarbonyl. Preference is given to cyclohexyloxycarbonyl.

$C_6$-$C_9$Cycloalkylcarbonyloxy is, for example, cyclohexylcarbonyloxy, cycloheptylcarbonyloxy, cyclooctylcarbonyloxy or cyclononylcarbonyloxy. Preference is given to cyclohexylcarbonyloxy.

$C_1$-$C_{12}$Alkyl-substituted benzoyloxy, which carries preferably from 1 to 3 alkyl groups, especially 1 or 2 alkyl groups, is, for example, o-, m- or p-methylbenzoyloxy, 2,3-dimethylbenzoyloxy, 2,4-dimethylbenzoyloxy, 2,5-dimethylbenzoyloxy, 2,6-dimethylbenzoyloxy, 3,4-dimethylbenzoyloxy, 3,5-dimethylbenzoyloxy, 2-methyl-6-ethylbenzoyloxy, 4-tert-butylbenzoyloxy, 2-ethylbenzoyloxy, 2,4,6-trimethylbenzoyloxy, 2,6-dimethyl-4-tert-butylbenzoyloxy or 3,5-di-tert-butylbenzoyloxy. Preferred substituents are $C_1$-$C_8$alkyl, especially $C_1$-$C_4$alkyl.

$C_2$-$C_{18}$Alkylene is a branched or unbranched radical, for example ethylene, propylene, trimethylene, tetramethylene, pentamethylene, hexamethylene, heptamethylene, octamethyllene, decamethylene, dodecamethylene or octadecamethylene. Preference is given to $C_1$-$C_{12}$alkylene, especially $C_1$-$C_8$alkylene.

$C_5$-$C_{12}$Cycloalkylene is a saturated hydrocarbon group having two free valences and at least one ring unit and is, for example, cyclopentylene, cyclohexylene, cycloheptylene, cyclooctylene, cyclononylene, cyclodecylene, cycloundecylene or cyclododecylene. Preference is given to cyclohexylene.

$C_8$-$C_{12}$Alkylene interrupted or terminated by cyclohexylene is, for example,

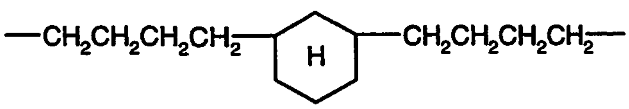

or

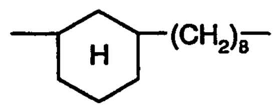

.

$C_4$-$C_{12}$Alkylene interrupted by oxygen, sulfur or by

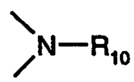

is, for example, $-CH_2CH_2-O-CH_2CH_2-$, $-CH_2CH_2-S-CH_2CH_2-$, $-CH_2CH_2-N(CH_3)-CH_2CH_2-$, $-CH_2CH_2-O-CH_2CH_2-O-CH_2CH_2-$, $-CH_2CH_2-(O-CH_2CH_2-)_2O-CH_2CH_2-$, $-CH_2CH_2-(O-CH_2CH_2-)_3O-CH_2CH_2-$, $-CH_2CH_2-(O-CH_2CH_2-)_4O-CH_2CH_2-$ or $-CH_2CH_2-S-CH_2CH_2-$.

A mono-, di- or tri-valent metal cation is preferably an alkali metal, alkaline earth metal or aluminium cation, for example, $Li^+$, $Na^+$, $K^+$, $Mg^{++}$, $Ca^{++}$ or $Al^{+++}$.

Fluorine-substituted $C_1$-$C_{18}$alkyl is a branched or unbranched radical, for example trifluoromethyl, pentafluoroethyl or hexafluoroisopropyl. A preferred meaning of $R_{20}$ is trifluoromethyl.

Preference is given to a process for the preparation of compounds of formula I wherein

$R_{11}$ is hydroxy,

$$\left[-O^{-}\ \frac{1}{r} M_b^{r+}\right],$$

$C_1$-$C_{18}$alkoxy,

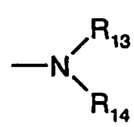

or a radical of formula IV;

$R_{13}$ and $R_{14}$ are each independently of the other hydrogen or $C_1$-$C_{18}$alkyl,

$M_b$ is an r-valent metal cation, and

r is 1, 2 or 3.

Of interest is a process for the preparation of compounds of formula I wherein $R_1$, $R_2$, $R_3$, $R_4$, $R_6$, $R_7$, $R_8$ and $R_9$ are each independently of the others hydrogen, halogen,$-SO_3H$, $-SO_3^-$ $M_a^+$, hydroxy, carboxy, cyano, nitro, $C_1$-$C_{18}$alkyl, fluorine- or $C_1$-$C_4$alkoxy-substituted $C_1$-$C_{18}$alkyl; $C_2$-$C_{18}$alkenyl, $C_7$-$C_9$phenylalkyl, unsubstituted or $C_1$-$C_4$alkyl-substituted phenyl; $C_5$-$C_8$cycloalkyl, $C_1$-$C_{18}$alkoxy, $C_1$-$C_{18}$alkylthio, $C_1$-$C_{18}$alkylsulfonyl, phenylsulfonyl, phe-

nylthio, $C_1$-$C_4$alkylamino, di($C_1$-$C_4$alkyl)amino, $C_1$-$C_{18}$alkanoyl, $C_1$-$C_{18}$alkoxycarbonyl, $C_1$-$C_{18}$alkanoyloxy, $C_1$-$C_{18}$alkanoylamino, $C_3$-$C_{18}$alkyl interrupted by oxygen, sulfur or by

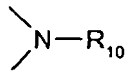 ;

$C_3$-$C_{18}$alkoxy interrupted by oxygen, sulfur or by

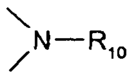 ;

$C_3$-$C_{18}$alkanoyloxy interrupted by oxygen, sulfur or by

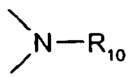 ;

$C_3$-$C_{18}$alkoxycarbonyl interrupted by oxygen, sulfur or by

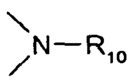 ;

$C_6$-$C_9$cycloalkoxycarbonyl, $C_6$-$C_9$cydoalkylcarbonyloxy, unsubstituted or $C_1$-$C_4$alkyl-substituted benzoyloxy; $-(CH_2)_p-COR_{11}$ or $-(CH_2)_qOH$; or, further, the radicals $R_6$ and $R_7$ or the radicals $R_7$ and $R_8$ or the radicals $R_8$ and $R_9$, together with the carbon atoms to which they are bonded, form a benzo ring, $R_3$ in addition is a radical of formula II and $R_6$ in addition is a radical of formula III,

$R_{10}$ is hydrogen or $C_1$-$C_6$alkyl,

$R_{11}$ is hydroxy,

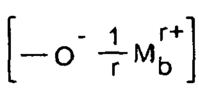 ,

$C_1$-$C_{12}$alkoxy,

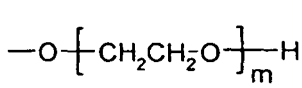 , 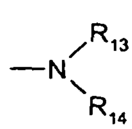

or a radical of formula IV,

$R_{12}$ is $-SO_2-$, $-SO_2-R_{16}-SO_2-$,

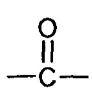 , 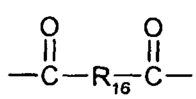 , 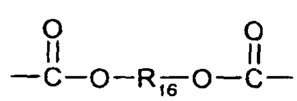

or

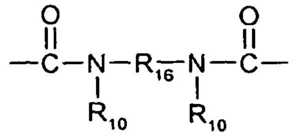 ,

$R_{13}$ and $R_{14}$ are each independently of the other hydrogen or $C_1$-$C_8$alkyl,
$R_{15}$ is

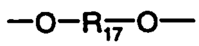 ,

$R_{16}$ is $C_2$-$C_{12}$alkylene or $C_5$-$C_{12}$cycloalkylene,
$R_{17}$ is $C_2$-$C_8$alkylene, or $C_4$-$C_{12}$alkylene interrupted by oxygen or by sulfur,
$R_{18}$ is halogen, nitro,

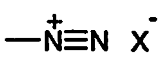 , 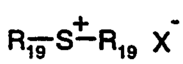 , 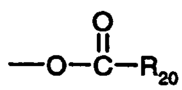 , 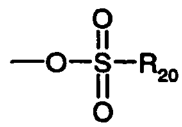

or $C_1$-$C_{12}$alkoxy,
$R_{19}$ is $C_1$-$C_{18}$alkyl, phenyl or $C_5$-$C_8$cycloalkyl,
$R_{20}$ is $C_1$-$C_{18}$alkyl, unsubstituted or $C_1$-$C_4$alkyl-, fluorine-, chlorine- or nitro-substituted phenyl; $C_5$-$C_8$cycloalkyl, or fluorine-substituted $C_1$-$C_{12}$alkyl,
$R_{21}$, $R_{22}$, $R_{23}$ and $R_{24}$ are each independently of the others hydrogen or $C_1$-$C_{12}$alkyl,
$R_{25}$ is $C_1$-$C_{12}$alkyl,
M is lithium, sodium, potassium, calcium,

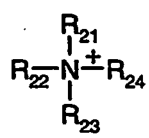

or $P^+(R_{25})_4$,
$M_a$ is sodium or potassium,
$M_b$ is sodium, potassium or calcium,
$X^-$ is chloride or bromide,
m is an integer from 1 to 15,
n is 1 or 2,
p is 0, 1 or 2,
q is 1, 2 or 3, and
r is 1 or 2.

Also of interest is a process for the preparation of compounds of formula I wherein
$R_1$ is hydrogen, chlorine, carboxy, nitro, $C_1$-$C_4$alkyl, trifluoromethyl or $C_1$-$C_4$alkoxy,
$R_2$ is hydrogen, chlorine, $-SO_3H$, $-SO_3^-M_a^+$, carboxy, cyano, nitro, $C_1$-$C_4$alkyl, trifluoromethyl, $C_1$-$C_4$alkoxy, benzyl, phenyl, cyclohexyl, $C_1$-$C_4$alkylsulfonyl, phenylsulfonyl, $C_1$-$C_8$alkanoyl, $C_1$-$C_8$alkoxycarbonyl, $C_1$-$C_8$alkanoyloxy, $C_3$-$C_8$alkyl interrupted by oxygen or by sulfur; $C_3$-$C_8$alkoxy interrupted by oxygen or by sulfur; $C_3$-$C_8$alkanoyloxy interrupted by oxygen or by sulfur; $C_3$-$C_8$alkoxycarbonyl interrupted by oxygen or by sulfur, cyclohexyloxycarbonyl, cyclohexylcarbonyloxy, or benzoyloxy,
$R_3$ is hydrogen or a radical of formula II,
$R_4$ is hydrogen, chlorine, carboxy, nitro, $C_1$-$C_4$alkyl, trifluoromethyl or $C_1$-$C_4$alkoxy,

$R_6$ is hydrogen, $C_1$-$C_{12}$alkyl, $C_7$-$C_9$phenylalkyl, phenyl, cyclohexyl, $C_1$-$C_{12}$alkoxy, $C_3$-$C_{12}$alkyl interrupted by oxygen or by sulfur; $C_3$-$C_{12}$alkoxy interrupted by oxygen or by sulfur; or a radical of formula III,
$R_7$ is hydrogen, chlorine, $C_1$-$C_4$alkyl or $C_1$-$C_4$alkoxy,
$R_8$ is hydrogen, $C_1$-$C_{12}$alkyl, $C_7$-$C_9$phenylalkyl, phenyl, cyclohexyl, $C_1$-$C_{12}$alkoxy, $C_3$-$C_{12}$alkyl interrupted by oxygen or by sulfur; $C_3$-$C_{12}$alkoxy interrupted by oxygen or by sulfur; or $-(CH_2)_p-COR_{11}$,
$R_9$ is hydrogen, chlorine, $C_1$-$C_4$alkyl or $C_1$-$C_4$alkoxy,
$R_{11}$ is hydroxy, $C_1$-$C_8$alkoxy,

$$-O-[CH_2CH_2O]_m-H$$

or a radical of formula IV,
$R_{12}$ is $-SO_2-$,

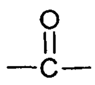 , 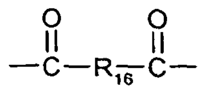 or 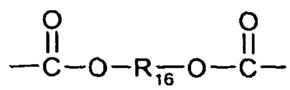 ,

$R_{15}$ is

$$-O-R_{17}-O- \quad ,$$

$R_{16}$ is $C_2$-$C_{18}$alkylene or $C_5$-$C_8$cycloalkylene,
$R_{17}$ is $C_2$-$C_8$alkylene, or $C_4$-$C_{12}$alkylene interrupted by oxygen,
$R_{18}$ is chlorine, bromine, iodine, nitro,

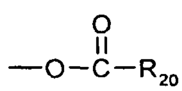 or 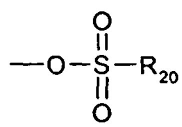 ,

$R_{20}$ is $C_1$-$C_8$alkyl, unsubstituted or fluorine-, chlorine- or nitro-substituted phenyl; or fluorine-substituted $C_1$-$C_4$alkyl,
M is lithium, sodium, potassium or calcium,
$M_a^+$ is sodium or potassium,
m is an integer from 1 to 15,
n is 1 or 2, and
p is 1 or 2.

Of special interest is a process for the preparation of compounds of formula I wherein $R_1$, $R_4$, $R_7$ and $R_9$ are hydrogen.

Likewise of special interest is a process for the preparation of compounds of formula I wherein $R_{18}$ is nitro, chlorine or bromine.

Of particular special interest is a process for the preparation of compounds of formula I wherein
$R_1$ is hydrogen or $C_1$-$C_4$alkyl,
$R_2$ is hydrogen, chlorine, $-SO_3H$, $-SO_3^-$ $M_a^+$, carboxy, cyano, nitro, $C_1$-$C_4$alkyl, trifluoromethyl, $C_1$-$C_4$alkoxy, $C_1$-$C_4$alkanoyl, $C_1$-$C_4$alkoxycarbonyl, $C_3$-$C_8$alkyl interrupted by oxygen; or $C_3$-$C_8$alkoxy interrupted by oxygen,
$R_3$ is hydrogen,
$R_4$ is hydrogen or $C_1$-$C_4$alkyl,
$R_6$ is hydrogen, $C_1$-$C_8$alkyl, $C_7$-$C_9$phenylalkyl, phenyl, cyclohexyl or a radical of formula III,
$R_7$ is hydrogen or $C_1$-$C_4$alkyl,
$R_8$ is hydrogen, $C_1$-$C_{12}$alkyl, $C_7$-$C_9$phenylalkyl, phenyl, cyclohexyl or $-(CH_2)_p-COR_{11}$,

$R_9$ is hydrogen or $C_1$-$C_4$alkyl,
$R_{11}$ is hydroxy, $C_1$-$C_8$alkoxy,

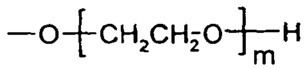

or a radical of formula IV,
$R_{15}$ is

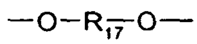 ,

$R_{17}$ is $C_4$-$C_{12}$alkylene interrupted by oxygen,
$R_{18}$ is chlorine, bromine, nitro,

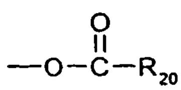 or 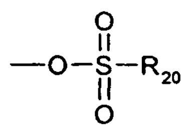 ,

$R_{20}$ is $C_1$-$C_4$alkyl, unsubstituted or fluorine-, chlorine- or nitro-substituted phenyl; or trifluoromethyl,
M is lithium, sodium or potassium,
$M_a^+$ is sodium or potassium,
m is an integer from 1 to 10,
n is 1, and
p is 2.

Special preference is given to a process for the preparation of compounds of formula I wherein
$R_1$ is hydrogen,
$R_2$ is hydrogen, chlorine, $-SO_3H$, $-SO_3^- M_a^+$, carboxy, cyano, nitro or trifluoromethyl,
$R_3$ is hydrogen,
$R_4$ is hydrogen,
$R_6$ is hydrogen, $C_1$-$C_5$alkyl, $\alpha,\alpha$-dimethylbenzyl or a radical of formula III,
$R_7$ is hydrogen,
$R_8$ is hydrogen, $C_1$-$C_8$alkyl or $-(CH_2)_p-COR_{11}$,
$R_9$ is hydrogen,
$R_{11}$ is $C_1$-$C_8$alkoxy,

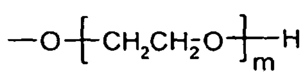

or a radical of formula IV,
$R_{15}$ is

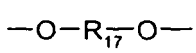 ,

$R_{17}$ is $C_4$-$C_{12}$alkylene interrupted by oxygen,
$R_{18}$ is nitro, chlorine or bromine,
M is lithium or sodium,
$M_a^+$ is sodium or potassium,

m is an integer from 1 to 10,
n is 1, and
p is 2.

Preferred reaction conditions of the process according to the invention are as follows:

The reaction can be carried out in the melt or in a solvent. Of special interest is a process for the preparation of compounds of formula I wherein the reaction is carried out in a solvent.

Suitable solvents are, for example, dipolar aprotic solvents, protic solvents, esters of aliphatic or aromatic carboxylic acids, ethers, halogenated hydrocarbons, aromatic solvents, amines and alkoxybenzenes.

Examples of dipolar aprotic solvents are dialkyl sulfoxides, for example dimethyl sulfoxide; carboxamides, for example formamide, dimethylformamide or N,N-dimethylacetamide; lactams, for example N-methylpyrrolidone; phosphoric amides, for example hexamethylphosphoric triamide; alkylated ureas, for example N,N'-dimethylethyleneurea, N,N'-dimethylpropyleneurea or N,N,N',N'-tetramethylurea; and nitriles, for example acetonitrile or benzonitrile.

Examples of protic solvents are polyalkylene glycols, for example polyethylene glycol; polyalkylene glycol monoethers, for example diethylene glycol monomethyl ether, and water, the latter on its own or in a single-phase or two-phase mixture with one or more of the solvents mentioned, it being possible also for phase transfer catalysts to be added, for example tetraalkylammonium salts, tetraalkylphosphonium salts or crown ethers. The same phase transfer catalysts can also be of use in solid/liquid form in the two-phase system.

Preferred esters of aliphatic or aromatic carboxylic acids are, for example, butyl acetate, cyclohexyl acetate and methyl benzoate.

Preferred ethers are, for example, dialkyl ethers, especially dibutyl ether, tetrahydrofuran, dioxane and (poly-) alkylene glycol dialkyl ethers.

Halogenated hydrocarbons are, for example, methylene chloride and chloroform.

Aromatic solvents are, for example, toluene, chlorobenzene and nitrobenzene.

Suitable amine solvents are, for example, triethylamine, tributylamine and benzyl-dimethylamine.

Preferred alkoxybenzenes are, for example, anisole and phenetole.

The process for the preparation of compounds of formula I can also be carried out in ionic or supercritical fluids, for example fluid carbon dioxide.

Of special interest is a process for the preparation of compounds of formula I wherein the reaction is carried out in a diplar aprotic solvent.

The reaction temperatures can be varied within wide limits but are so selected that satisfactory conversion occurs, such temperatures preferably being from 10° to 180°C, especially from 20° to 150°C. The reaction is preferably so carried out that the intermediate of formula X

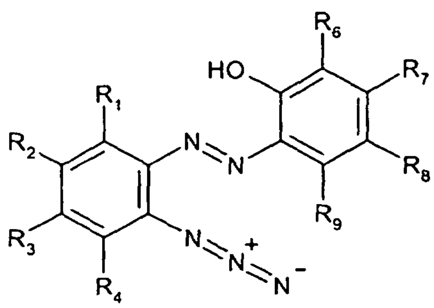

(X)

is not formed at all, or at most is formed only in a small amount and immediately reacts further to form the compound of formula I.

Preference is given to a process for the preparation of compounds of formula I wherein the molar ratio of the amount of compound of formula V to the amount of azide compound of formula IX is from 1 : 1 to 1 : 3, especially from 1 : 1 to 1 : 2, e.g. from 1 : 1 to 1 : 1.3. When functional side groups that are also able to react with azide are present, the excess of the azide compound of formula IX is increased accordingly.

When $R_{18}$ is a halogen atom, for example, chlorine, bromine or iodine, the reaction can be accelerated by the addition of a suitable catalyst. Such catalysts include, for example, copper(I) or copper(II) salts or other transition metal salts, based, for example, on iron, cobalt, nickel, palladium, platinum, gold or zinc. Instead of transition metal salts, the anions of which can be varied within wide limits, it is also possible to use metal complexes and metal complex salts of the same metals as catalysts. Preference is given to the use of copper(I) and copper(II) chlorides, bromides and iodides, and special preference to the use of copper(I) bromide.

Accordingly, there is also of special interest a process for the preparation of compounds of formula I wherein the reaction is carried out in the presence of a catalyst.

The catalyst is advantageously used in an amount of from 0.01 to 10 % by weight, especially from 0.1 to 5 % by weight, e.g. from 0.1 to 5 % by weight, based on the weight of the compound of formula V employed.

The reaction can also be carried out in the presence of an additional base or in the presence of an alkaline pH buffer system. Suitable pH buffer systems include, for example, alkali metal or alkaline earth metal hydroxides; alkali metal or alkaline earth metal alcoholates; alkali metal or alkaline earth metal carboxylates, for example acetates or carbonates; alkali metal or alkaline earth metal phosphates; tertiary amines, for example triethylamine or tributylamine; and unsubstituted or substituted pyridines.

The starting materials of formula V can be used in the form of pure substances or in the form of crude solutions that comprise the compounds of formula V. The compounds of formula I can also be prepared in a so-called one-pot process. In that process the compounds of formula V are prepared *in situ* and are reacted with a compound of formula IX, without being isolated, to form the compounds of formula I.

Working up of the reaction mixture is advantageously carried out by evaporating off the solvent at normal pressure or *in vacuo.* The reaction mixture can also be diluted with water, extracted with an organic solvent, for example toluene, and then concentrated by evaporation. The residue comprising the compounds of formula I is purified by customary known methods, for example recrystallisation, precipitation, crystallisation, distillation at normal pressure or *in vacuo,* chromatography on silica gel or aluminium oxide, or adsorption of the polar impurities on solid phases, for example fuller's earths, activated carbon or Hyflo. The choice of working-up method depends on the physical properties of the compound of formula I and of any secondary products.

Most of the starting compounds of formula V are known from the literature or can be prepared analogously to the procedures described in Examples 9a, 10a, 12a and 13a.

The compounds of formula I are UV absorbers and are suitable as stabilisers for organic materials. Many of them are obtainable commercially from Ciba Spezialitätenchemie AG, for example Tinuvin 327 (RTM) (compound 109, Table 1); Tinuvin 328 (RTM) (compound 106, Table 1); Tinuvin 343 (RTM) (compound 103, Table 1); and Tinuvin P (RTM) (compound 104, Table 1).

The following Examples illustrate the invention further. Parts or percentages relate to weight.

<u>Reference Example 1</u>: Preparation of 2-phenyl-4,5-benzo-1,2,3-triazole (compound 101, Table 1).

1.0 g (4.40 mmol) of 2-nitro-azobenzene (compound 201, Table 2) and 0.34 g (5.28 mmol) of sodium azide are stirred for 10 hours at 125˚C in 5 ml of dimethyl sulfoxide. After the conversion has taken place, the mixture is cooled and toluene and water are added. The organic phase is washed repeatedly with water and then with 2N hydrochloric acid solution, dried over sodium sulfate and concentrated by evaporation *in vacuo.* 0.82 g (95 %) of 2-phenyl-4,5-benzo-1,2,3-triazole (compound 101, Table 1), m.p. 106-107˚C (Lit. 108-109˚C, P. Spagnolo et al., J. Chem. Soc., Perkin Trans. I 1988, 2615) is obtained.

<u>Reference Example 2</u>: Preparation of compound 102 (Table 1).

0.40 g (1.53 mmol) of 1-chloro-4-nitro-2-(phenylazo)-benzene (compound 202, Table 2) is dissolved in 2 g of N-methylpyrrolidone, and 0.12 g (1.83 mmol) of sodium azide is added. The mixture is stirred for 4 hours at 25˚C. After the conversion has taken place, the mixture is cooled and toluene and water are added. The organic phase is washed repeatedly with water and then with 2N hydrochloric acid solution, dried over sodium sulfate and concentrated using a vacuum rotary evaporator. 0.31 g (86 %) of compound 102 (Table 1), m.p. 170-172˚C (Lit. 175-177˚C, P. G. Houghton et al., J. Chem. Soc., Perkin Trans I 1985, 1471) is obtained.

<u>Example 3</u>: Preparation of compound 103 (Table 1) starting from compound 203 (Table 2).

1.0 g (2.81 mmol) of 2-(2-butyl)-4-tert-butyl-6-(2-nitrophenylazo)-phenol (compound 203, Table 2) is stirred for 4 hours at 125˚C with 5 ml of dimethylformamide and 0.24 g (3.69 mmol) of sodium azide. After the reaction has taken place, the mixture is taken up in water and extracted with THF/toluene (1:1). The organic phases are washed three times with water and once with saturated sodium chloride solution, dried over sodium sulfate and concentrated using a vacuum rotary evaporator. 0.88 g (92 %) of 2-(2-butyl)-4-tert-butyl-6-(4,5-benzo-1,2,3-triazol-2-yl)-phenol (compound 103, Table 1), m.p. 81-84˚C, is obtained.

The use of dimethyl sulfoxide, N,N-dimethylacetamide or N-methylpyrrolidone instead of dimethylformamide under otherwise identical conditions produces very similar results.

When the reaction is carried out in dimethylformamide under otherwise identical conditions with the addition of 0.52 g (2.81 mmol) of tributylamine, the reaction is complete after 4 hours at 160˚C and the yield of compound 103

(Table 1) is 0.85 g (89 %).

When the reaction is carried out in dimethylformamide under otherwise identical conditions with the addition of 2.03 g (6.75 mmol) of polyethylene glycol 300, the reaction is complete after 4 hours at 160˚C and the yield of compound 103 (Table 1) is 0.83 g (87 %).

Example 4: Preparation of compound 103 (Table 1) starting from compound 204 (Table 2).

1.0 g (2.90 mmol) of 2-(2-butyl)-4-tert-butyl-6-(2-chloro-phenylazo)-phenol (compound 204, Table 2), 5 g of N, N-dimethylformamide, 0.226 g (3.48 mmol) of sodium azide and 4.2 mg (0.029 mmol; 1 mol %) of copper(I) bromide are combined and stirred for 4 hours at 80˚C. After the conversion has taken place, the mixture is cooled and toluene and water are added. The organic phase is washed repeatedly with water and then with 2N hydrochloric acid solution, dried over sodium sulfate, filtered over silica gel and concentrated using a vacuum rotary evaporator. 0.90 g (92 %) of 2-(2-butyl)-4-tert-butyl-6-(4,5-benzo-1,2,3-triazol-2-yl)-phenol (compound 103, Table 1), m.p. 81-84˚C, is obtained.

The use of ethylene glycol monobutyl ether instead of dimethylformamide under similar conditions produces comparable results, complete conversion being achieved after 4 hours at 120˚C.

The use of butyl acetate instead of dimethylformamide under similar conditions produces comparable results, almost complete conversion being achieved after stirring for one day at reflux at approximately 125˚C.

Example 5: Preparation of compound 103 (Table 1) starting from compound 205 (Table 2).

1.0 g (2.57 mmol) of 2-(2-butyl)-4-tert-butyl-6-(2-bromo-phenylazo)-phenol (compound 205, Table 2), 5 g of N,N-dimethylformamide, 0.2 g (3.08 mmol) of sodium azide and 3.7 mg (0.0257 mmol; 1 mol %) of copper(I) bromide are combined and stirred for 2 hours at 80˚C. After the conversion has taken place, the mixture is cooled and toluene and water are added. The organic phase is washed repeatedly with water and then with 2N hydrochloric acid solution, dried over sodium sulfate, filtered over silica gel and concentrated using a vacuum rotary evaporator. 0.80 g (92 %) of 2-(2-butyl)-4-tert-butyl-6-(4,5-benzo-1,2,3-triazol-2-yl)-phenol (compound 103, Table 1), m.p. 81-84˚C, is obtained.

Example 6: Preparation of compound 104 (Table 1).

1.0 g (3.89 mmol) of 4-methyl-2-(2-nitrophenylazo)-phenol (compound 206, Table 1) is stirred for 4 hours at 130˚C with 5 ml of N,N-dimethylacetamide and 0.30 g (4.66 mmol) of sodium azide. After the reaction has taken place, the mixture is taken up in water and extracted with toluene. The organic phases are washed five times with water and once with 2N hydrochloric acid solution, dried over sodium sulfate and concentrated by evaporation *in vacuo* at 80˚C. 0.81 g (86 %) of 4-methyl-2-(4,5-benzo-1,2,3-triazol-2-yl)-phenol (compound 104, Table 1), m.p. 128-132˚C (Lit. 131.5-133˚C; J. H. Hall, J. Org. Chem. 1986, 33, 2954).

The use of dimethyl sulfoxide, dimethylformamide or N-methylpyrrolidone instead of dimethylacetamide under otherwise identical conditions produces very similar results.

Example 7: Preparation of compound 105 (Table 1).

1.0 g (2.11 mmol) of 2-cumyl-4-(1,1,3,3-tetramethyl-butyl)-6-(2-nitrophenylazo)-phenol (compound 207, Table 2) is stirred for approximately 4 hours at 130˚C with 5 ml of N,N-dimethylacetamide and 0.165 g (2.53 mmol) of sodium azide. After the reaction has taken place, the mixture is taken up in water and extracted with toluene. The organic phases are washed five times with water and once with 2N hydrochloric acid solution, dried over sodium sulfate and concentrated using a vacuum rotary evaporator. 0.94 g (97 %) of 2-cumyl-4-(1,1,3,3-tetramethyl-butyl-6-(4,5-benzo-1,2,3-triazol-2-yl)-phenol (compound 105, Table 1), m.p. 128-132˚C, is obtained

Example 8: Preparation of compound 106 (Table 1).

58.5 g (0.15 mol) of 2,4-bis-(1,1-dimethylpropyl-)-6-(2-nitrophenylazo)-phenol (compound 208, Table 2) are stirred for 13 hours at 130˚C with 120 g of dimethylformamide and 10.8 g (0.164 mol) of sodium azide. After the reaction has taken place, dimethylformamide is distilled off *in vacuo* and the mixture is taken up in xylene. After the addition of water, the aqueous phase is separated off and the organic phase is dried over sodium sulfate and concentrated using a vacuum rotary evaporator. Crystallisation from methanol yields 49.5 g (93.9 %) of 2,4-bis-(1,1-dimethylpropyl)-6-(4,5-benzo-1,2,3-triazol-2-yl)-phenol (compound 106, Table 1), m.p. 80-88˚C.

Example 9: Preparation of compound 107 (Table 1).

a) Preparation of compound 209 (Table 2).

25.25 g (0.20 mol) of 2-chloroaniline are introduced in the course of 20 minutes at 20°C, with stirring, into 53.6 g of 32 % hydrochloric acid, a suspension being formed. After the addition of 10 ml of water and cooling to from 10 to 0°C, 34.5 g of a 40 % solution of sodium nitrite in water are added dropwise in the course of 30 minutes. The resulting diazonium salt solution is stirred for a further 30 minutes at -10°C. After the addition of 0.06 g of urea to eliminate excess nitrite, undissolved constituents are filtered off. The solution of the diazonium salt is so added in the course of 45 minutes, with cooling, to a solution of 6.96 g (0.174 mol) of sodium hydroxide and 41.15 g (0.174 mol) of β-(4-tert-butyl-4-hydroxyphenyl)-propionic acid methyl ester in 150 ml of methanol that the temperature remains below 0°C. When the addition of the diazonium salt is complete, the mixture is gradually brought to 25°C and stirred for 90 minutes to complete the reaction. After the introduction of 250 ml of toluene with stirring, and the addition of 30 ml of water, the aqueous phase is separated off and the organic phase is washed with 100 ml of water. The organic phase is dried over sodium sulfate and concentrated using a vacuum rotary evaporator. Crystallisation of the residue from isopropanol yields 36.2 g (50 %) of compound 209, (Table 2), m.p. 88-91 °C.

b) Preparation of compound 107 (Table 1).

9.0 g (24.0 mmol) of compound 209 (Table 2, prepared according to Example 9a) are stirred for 2 hours at 90°C with 30 ml of dimethylformamide, 2.1 g (32.0 mmol) of sodium azide und 36 mg of copper(I) bromide. After the reaction has taken place, the mixture is cooled, taken up in 50 ml of water and extracted with 50 ml of toluene. The organic phases are washed twice with water, once with 2N hydrochloric acid and once with saturated sodium chloride solution, dried over sodium sulfate and concentrated using a vacuum rotary evaporator. Crystallisation of the residue from toluene/methanol yields 6.41 g (76 %) of compound 107, (Table 1), m.p. 119-124°C.

Analogously to Example 9b, compound 107 (Table 1) is likewise obtained using compound 213 (Table 2) instead of compound 209 (Table 2).

Example 10: Preparation of compound 108 (Table 1) with the isolation of compound 210 (Table 2).

a) Preparation of compound 210 (Table 2).

39.12 g (0.2 mol) of 3-amino-4-chlorobenzotrifluoride are introduced in the course of 100 minutes at from 0 to 5°C, with stirring, into a mixture of 80 g of water and 54 g of 32 % hydrochloric acid. After further stirring for 30 minutes at 0°C, 34.5 g (0.2 mol) of a 40 % solution of sodium nitrite in water are added dropwise in the course of 75 minutes. The suspension is stirred for a further 45 minutes at 0°C. The resulting suspension of the diazonium salt is so added in the course of 45 minutes, with cooling, to a solution of 9.04 g (0.226 mol) of sodium hydroxide and 42.6 g (0.1244 mol) of 2-cumyl-4-(1,1,3,3-tetramethylbutyl)-phenol in 20 ml of xylene and 140 g of methanol that the temperature remains below 5°C. After half of the addition, a further 20 g of xylene and 140 g of methanol are added. When the addition of the diazonium salt is complete, the mixture is gradually brought to 25°C and stirred for 18 hours to complete the reaction. After removal of the aqueous phase, the organic phase is neutralised with acetic acid and washed with 100 ml of water. The organic phase is dried over sodium sulfate and concentrated using a vacuum rotary evaporator. Crystallisation of the residue from isopropanol yields 42.3 g of 2-cumyl-4-(1,1,3,3-tetramethyl-butyl)-6-(2-chloro-5-trifluoromethyl-phenylazo)-phenol (compound 210, Table 2), m.p. 120-127°C.

b) Preparation of compound 108 (Table 1).

9.0 g (16.9 mmol) of 2-cumyl-4-(1,1,3,3-tetramethyl-butyl)-6-(2-chloro-5-trifluoromethyl-phenylazo)-phenol (compound 210, Table 2, prepared according to Example 10a) are stirred for 2 hours at 120°C with 30 ml of dimethylformamide, 1.7 g (16 mmol) of triethylamine and 1.5 g (22.2 mmol) of sodium azide. After the reaction has taken place, the mixture is cooled, taken up in 50 ml of water and extracted with 50 ml of toluene. The organic phase is washed four times with water, dried over sodium sulfate and concentrated using a vacuum rotary evaporator. Crystallisation of the residue from isopropanol/methanol = 3:1 yields 6.8 g (79 %) of 2-cumyl-4-(1,1,3,3-tetramethyl-butyl)-6-[(5'-trifluoromethyl)-4,5-benzo-1,2,3-triazol-2-yl]-phenol (compound 108, Table 1), m.p. 92-95°C.

Example 11: Preparation of compound 108 (Table 1) without the isolation of compound 210 (Table 2).

39.12 g (0.20 mol) of 3-amino-4-chlorobenzotrifluoride are diazotized as described in Example 10a, but at from

0 to -10°C. The resulting suspension is stirred for 70 minutes at from -5 to -10°C. The resulting suspension of the diazonium salt is so added in the course of 50 minutes, with cooling, to a solution of 14.4 g (0.366 mol) of sodium hydroxide and 68.32 g (0.20 mol) of 2-cumyl-4-(1,1,3,3-tetramethybutyl)-phenol in 30 ml of xylene and 170 ml of methanol that the temperature remains below -5°C. When the addition of the diazonium salt is complete, the mixture is gradually brought to 25°C and is further stirred for 120 minutes. After removal of the aqueous phase, 200 ml of xylene are added and the organic phase is neutralised with acetic acid, washed with 100 ml of water and concentrated using a vacuum rotary evaporator. After concentration of the organic phase *in vacuo,* 118.6 g of the crude product of compound 210 (Table 2) are obtained. 117.6 g of that crude product are heated to 135°C in the course of 90 minutes with 300 ml of DMF, 22.4 g (0.22 mol) of triethylamine and 18.7 g (0.28 mol) of sodium azide and stirring is carried out for approximately 4.5 hours at from 130 to 135°C. After the reaction has taken place, the mixture is cooled, diluted with 500 ml of water and extracted with 500 ml of toluene. The organic phase is washed four times with water, once with 2N hydrochloric acid and once with saturated sodium chloride solution, dried over sodium sulfate and concentrated using a vacuum rotary evaporator. Crystallisation of the residue from xylene/methanol yields 58.9 g (58 %) of 2-cumyl-4-(1,1,3,3-tetramethyl-butyl)-6-[(5'-trifluoromethyl)-4,5-benzo-1,2,3-triazol-2-yl]-phenol (compound 108, Table 1), m.p. 92-95°C.

Example 12: Preparation of compound 109 (Table 1) with the isolation of compound 211 (Table 2).

a) Preparation of compound 211 (Table 2).

32.44 g (0.20 mol) of molten 2,5-dichloroaniline are introduced in the course of 40 minutes at 50°C, with stirring, into 53.6 g of 32 % hydrochloric acid. After the addition of 20 ml of water and cooling to 0°C, 34.5 g of a 40 % solution of sodium nitrite in water are added dropwise in the course of 80 minutes. The suspension is stirred at 0°C for 85 minutes. The resulting suspension of the diazonium salt is so added in the course of 50 minutes, with cooling, to a solution of 9.04 g (0.226 mol) of sodium hydroxide and 25.7 g (0.1244 mol) of 2,4-di-tert-butylphenol in 150 ml of methanol that the temperature remains below 0°C. When the addition of the diazonium salt is complete, the mixture is gradually brought to 25°C and further stirred for 90 minutes. After the addition of 400 ml of toluene, the aqueous phase is separated off and the organic phase is neutralised with acetic acid, washed with water, dried over sodium sulfate and concentrated using a vacuum rotary evaporator. Crystallisation of the residue from a 1:1 mixture of xylene/methanol yields 28.1 g (64 %) of 2,4-di-tert-butyl-6-(2,5-dichlorophenylazo)-phenol (compound 211, Table 2), m.p. 121-126°C.

b) Preparation of compound 109 (Table 1).

9.0 g (23.7 mmol) of 2,4-di-tert-butyl-6-(2,5-dichlorophenylazo)-phenol (compound 211, Table 2, prepared according to Example 12a) are stirred for 4 hours at 80°C with 30 ml of dimethylformamide, 2.0 g (30.8 mmol) of sodium azide, 2.43 g of triethylamine and 0.034 g of copper(I) bromide. After the reaction has taken place, the mixture is taken up in water and extracted with toluene. The organic phases are washed four times with water, once with 2N hydrochloric acid and once with saturated sodium chloride solution, dried over sodium sulfate and concentrated using a vacuum rotary evaporator. Crystallisation of the residue from toluene/methanol yields 7.13 g (84 %) of 2,4-di-tert-butyl-6-(5'-chloro-4,5-benzo-1,2,3-triazol-2-yl)-phenol (compound 109, Table 1), m.p. 149-153°C.

Example 13: Preparation of compound 109 (Table 1) with the isolation of compound 212 (Table 2).

a) Preparation of compound 212 (Table 2).

32.44 g (0.20 mol) of molten 2,4-dichloroaniline are introduced in the course of 40 minutes at 50°C, with stirring, into 53.6 g of 32 % hydrochloric acid. After the addition of 40 ml of water and cooling to 0°C, 34.5 g of a 40 % solution of sodium nitrite in water are added dropwise in the course of 65 minutes. The suspension is stirred for 85 minutes at 0°C. The resulting suspension of the diazonium salt is so added in the course of 85 minutes, with cooling, to a solution of 9.04 g (0.226 mol) of sodium hydroxide and 25.7 g (0.1244 mol) of 2,4-di-tert-butylphenol in 150 ml of methanol that the temperature remains below 0°C. When the addition of the diazonium salt is complete, the mixture is gradually brought to 25°C and stirred for a further 90 minutes. After decanting off the liquid phase, the solid product is taken up in 200 ml of toluene and washed with 100 ml of water. The organic phase is dried over sodium sulfate and concentrated using a vacuum rotary evaporator. Crystallisation of the residue from a 1:1 mixture of xylene/methanol yields 31.3 g (70 %) of 2,4-di-tert-butyl-6-(2,4-dichlorophenylazo)-phenol (compound 212, Table 2), m.p.164-168°C.

b) Preparation of compound 109, Table 1).

9.0 g (23.7 mmol) of 2,4-di-tert-butyl-6-(2,4-dichlorophenylazo)-phenol (compound 212, Table 2, prepared

according to Example 13a), are reacted with sodium azide as described in Example 12b. Crystallisation of the residue from toluene/methanol yields 6.55 g (77 %) of 2,4-di-tert-butyl-6-(5'-chloro-4,5-benzo-1,2,3-triazol-2-yl)-phenol (compound 109, Table 1), m.p. 149-153˚C.

Example 14: Preparation of compound 110 (Table 1).

a) Preparation of compound 214 (Table 2).

10.8 g (63 mmol) of 2-chloro-5-nitroaniline are triturated with 0.2 g of Steramid [N,N'-ethylene-bis-stearic amide; C.A. Reg. No. 110-30-5]. The mixture is then introduced in portions into a solution of 16.5 ml of concentrated hydrochloric acid in 10 ml of water and stirring is carried out for 16 hours. After cooling to from -10 to -15˚C, 16 ml of a 4N solution of sodium nitrite in water are added dropwise in the course of from 1 to 2 hours. The resulting diazonium salt solution is stirred for 10 minutes at -10˚C. 2.2 g (54 mmol) of sodium hydroxide beads are dissolved, with stirring, in a solution of 18.5 g (54 mmol) of 4-(1,1,3,3 tetramethylbutyl)-2-cumyl-phenol (95 % purity) in 140 ml of methanol and 20 ml of xylene. 4.0 g (54 mmol) of calcium hydroxide are then added and the resulting suspension is cooled to lower -15˚C. Then, at from -15 to -5˚C, the diazonium salt solution is added dropwise within a period of 30 minutes. The red suspension is gradually brought to 25˚C and stirred overnight to complete the reaction. After the introduction of 150 ml of toluene with stirring and the addition of 100 ml of water, the aqueous phase is separated off and the organic phase is washed four times with 100 ml of water each time. The organic phases are combined and concentrated using a vacuum rotary evaporator. Crystallisation of the residue from xylene/methanol yields 16.2 g (59 %) of 2-(2-chloro-5-nitrophenylazo)-6-(1-methyl-1-phenylethyl)-4-(1,1,3,3-tetramethylbutyl)-phenol (compound 214, Table 2). M.p. 153 - 156˚C.

b) Preparation of compound 110 (Table 1).

10.16 g (20 mmol) of 2-(2-chloro-5-nitro-phenylazo)-6-(1-methyl-1-phenyl-ethyl)-4-(1,1,3,3-tetramethylbutyl)-phenol [compound 214 (Table 2), prepared according to Example 14a], are stirred for approximately 4 hours at 40˚C with 60 ml of dimethylformamide and 1.69 g (26 mmol) of sodium azide. After the reaction has taken place, the mixture is taken up in water and extracted with methylene chloride. The organic phases are washed repeatedly with water and concentrated by evaporation. Crystallisation of the residue from isopropanol/toluene yields 8.53 g (88 %) of 2-(1-methyl-1-phenyl-ethyl)-6-(5-nitro-benzotriazol-2-yl)-4-(1,1,3,3-tetramethyl-butyl)-phenol (compound 110, Table 1). M.p. 153 - 156˚C.

Example 15: Preparation of compound 111 (Table 1).

a) Preparation of compound 215 (Table 2).

45 g of 32 % hydrochloric acid are rapidly added to a solution of 11.3 g (66 mmol) of 3-amino-4-chlorobenzoic acid in 10.9 g of 30 % sodium hydroxide solution and 40 ml of water. After subsequently stirring for one hour, the mixture is cooled to from -5 to 0˚C. 12.5 ml of an aqueous 40 % sodium nitrite solution (approximately 65 mmol of $NaNO_2$) are then metered in at from -5 to 0˚C. After subsequently stirring for one hour, excess nitrite is eliminated using a small amount of sulfamic acid. 3.04 g (76 mmol) of sodium hydroxide beads are dissolved, with stirring, in a solution of 26.0 g (76 mmol) of 4-(1,1,3,3-tetramethylbutyl)-2-cumyl-phenol (95 % purity) in 70 ml of methanol and 10 ml of xylene. 5.63 g (76 mmol) of calcium hydroxide are then added and the resulting suspension is cooled to 0˚C. The diazonium salt solution is then added dropwise thereto at from 0 to 5˚C. In parallel, approximately 10 g of calcium hydroxide and 20 ml of 30 % sodium hydroxide solution are metered in in order to maintain an alkaline pH. When the addition is complete, the mixture is gradually brought to 25˚C and stirred overnight to complete the reaction. After the introduction, with stirring, of 50 ml of water, 50 ml of 32 % hydrochloric acid, 100 ml of toluene and 200 ml of ethyl acetate, the aqueous phase is separated off and the organic phase is washed twice with 100 ml of water. The organic phases are combined and concentrated using a vacuum rotary evaporator. Crystallisation of the residue from methanol/hexane yields 12.1 g (36 %) of 4-chloro-3-[2-hydroxy-3-(1-methyl-1-phenyl-ethyl)-5-(1,1,3,3-tetramethyl-butyl)-phenylazo]-benzoic acid (compound 215, Table 2). M.p. 218-220˚C.

b) Preparation of compound 111 (Table 1).

5.07 g (10 mmol) of 4-chloro-3-[2-hydroxy-3-(1-methyl-1-phenyl-ethyl)-5-(1,1,3,3-tetramethylbutyl)-phenylazo]-benzoic acid [compound 215 (Table 2), prepared according to Example 15a] are stirred for approximately 4 hours at 140˚C with 35 ml of dimethylformamide and 0.85 g (13 mmol) of sodium azide. After the reaction has taken place, the mixture is taken up in water, 5 ml of acetic acid are added and extraction is carried out with toluene. The organic

phases are washed repeatedly with water and concentrated using a vacuum rotary evaporator. Crystallisation of the residue from isopropanol yields 2.5 g (52 %) of 2-[2-hydroxy-3-(1-methyl-1-phenyl-ethyl)-5-(1,1,3,3-tetramethyl-butyl)-phenyl]-2H-benzotriazole-5-carboxylic acid (compound 111, Table 1). M.p. 219-221 ˚C.

Example 16: Preparation of compound 112 (Table 1).

a) Preparation of compound 216 (Table 2).

5.0 g (32.7 mmol) of 3-amino-4-chlorobenzonitrile are stirred, in portions, into 60 ml of water at 95˚C. 30 ml of 32 % hydrochloric acid are then added dropwise, and the mixture is cooled to room temperature and stirred for 16 hours to complete the reaction. After cooling to from -10 to -15˚C, 9.0 ml of a 4N solution of sodium nitrite in water are metered in in the course of 40 minutes. The resulting diazonium salt solution is stirred for 30 minutes at -10˚C. 2.2 g (54 mmol) of sodium hydroxide beads are dissolved, with stirring, in a solution of 5 g (0.54 mmol) of 4-(1,1,3,3 tetramethylbutyl)-2-cumyl-phenol (95 % purity) in 70 ml of methanol and 10 ml of xylene. After the solution has been cooled to < -15˚C, the diazonium salt solution is added dropwise thereto within a period of 100 minutes at from -15 to -5˚C. During the addition an alkaline pH is maintained by metering in approximately 30 ml of 30 % sodium hydroxide solution in parallel. When the addition is complete, 50 ml of xylene are added. The red suspension is gradually brought to 25˚C and stirred overnight to complete the reaction. After the introduction of 150 ml of ethyl acetate with stirring, and the addition of 100 ml of water and 5 ml of acetic acid, the aqueous phase is separated off and the organic phase is washed three times with 100 ml of water each time. The organic phases are combined and concentrated using a vacuum rotary evaporator. Crystallisation of the residue from methanol yields 9.4 g (59 %) of 4-chloro-3-[2-hydroxy-3-(1-methyl-1-phenyl-ethyl)-5-(1,1,3,3-tetramethyl-butyl)-phenylazo]-benzonitrile (compound 216, Table 2). M.p. 190-191 ˚C.

b) Preparation of compound 112 (Table 1).

4.88 g (10 mmol) of 4-chloro-3-[2-hydroxy-3-(1-methyl-1-phenyl-ethyl)-5-(1,1,3,3-tetramethylbutyl)-phenylazo]-benzonitrile [compound 216 (Table 2), prepared according to Example 16a] are stirred for one hour at 120˚C with 12.5 ml of dimethylformamide and 0.85 g (13 mmol) of sodium azide. After the reaction has taken place, the mixture is taken up in water and extracted with toluene. The organic phases are washed repeatedly with water, combined and concentrated using a vacuum rotary evaporator. Crystallisation of the residue from hexane yields 3.6 g (77 %) of 2-[2-hydroxy-3-(1-methyl-1-phenyl-ethyl)-5-(1,1,3,3-tetramethyl-butyl)-phenyl]-2-benzotriazole-5-carbonitrile (compound 112, Table 1). M.p. 199 - 201 ˚C

Example 17: Preparation of compound 113 (Table 1).

a) Preparation of compound 218 (Table 2).

27.4 g (0.132 mol) of 2-chloroaniline-5-sulfonic acid are dissolved in 21.8 g of 30 % sodium hydroxide solution and 80 ml of water. 90 ml of 32 % hydrochloric acid are then rapidly added and the suspension is stirred for one hour. After cooling to from 0 to -5˚C, 32.5 ml of a 4N solution of sodium nitrite in water are metered in in the course of 60 minutes. After subsequently stirring for one hour at from 0 to -5˚C, excess nitrite is eliminated using a small amount of sulfamic acid. 6.08 g (0.152 mol) of sodium hydroxide beads are dissolved, with stirring, in a solution of 45.1 g (0.132 mol) of 4-(1,1,3,3 tetramethylbutyl)-2-cumyl-phenol (95 % purity) in 14 ml of methanol and 20 ml of xylene. 11.3 g (0.152 mol) of calcium hydroxide are then added and the resulting suspension is cooled to 0˚C. The diazonium salt solution is then added dropwise thereto at from 0 to 5˚C. In parallel, approximately 10 g of calcium hydroxide and 30 ml of 30 % sodium hydroxide solution are metered in to maintain an alkaline pH. When the addition is complete, the mixture is gradually brought to 25˚C and stirred overnight to complete the reaction. After the addition of 100 ml of water and 75 ml of 32 % hydrochloric acid, 300 ml of xylene and 150 ml of ethyl acetate are stirred in. The aqueous phase is separated off and the organic phase is washed three times with 100 ml of water each time. The organic phases are combined and concentrated using a vacuum rotary evaporator. 68.3 g of crude 4-chloro-3-[2-hydroxy-3-(1-methyl-1-phenyl-ethyl)-5-(1,1,3,3-tetramethyl-butyl)-phenylazo]-benzenesulfonic acid (compound 217, Table 2) are obtained. M.p. 182˚C (decomposition). 300 ml of water and 30 ml of 30 % sodium hydroxide solution are added to 67.3 g (0.124 mol) of that compound, and the mixture is heated to from 80 to 85˚C and stirred for 0.5 hour at that temperature. After cooling to room temperature, the supernatant aqueous phase is decanted off and the residue is dissolved hot in 150 ml of water and 150 ml of ethanol. The product that crystallises out on cooling is dried *in vacuo.* 48.0 g (64 %) of 4-chloro-3-[2-hydroxy-3-(1-methyl-1-phenyl-ethyl)-5-(1,1,3,3-tetramethylbutyl)-phenylazo]-benzenesulfonic acid sodium salt (compound 218, Table 2) are obtained. M.p. 243 - 245˚C.

b) Preparation of compound 113 (Table 1).

5.65 g (10 mmol) of 4-chloro-3-[2-hydroxy-3-(1-methyl-1-phenyl-ethyl)-5-(1,1,3,3-tetramethylbutyl)-phenylazo]-benzenesulfonic acid sodium salt [compound 218 (Table 2), prepared according to Example 17a] are stirred for 8 hours at 160˚C with 20 ml of N-methyl-2-pyrrolidone and 0.85 g (13 mmol) of sodium azide. After the reaction has taken place, 50 ml of toluene, 30 ml of water and three drops of 2N hydrochloric acid are added. The phases are separated and the organic phase is washed with 30 ml of water. The aqueous phases are re-extracted with 30 ml of toluene. The organic phases are combined and concentrated using a vacuum rotary evaporator. Crystallisation of the residue from isopropanol yields 3.9 g (71 %) of 2-[2-hydroxy-3-(1-methyl-1-phenyl-ethyl)-5-(1,1,3,3-tetramethyl-butyl)-phenyl]-2H-benzotriazole-5-sodium sulfonate (compound 113, Table 1). M.p. 361˚C (decomposition).

Example 18: Preparation of compound 114 (Table 1).

0.30 g (0.415 mmol) of 2,2'-methylene-bis[6-(2-nitrophenyl)azo-4-(1,1,3,3-tetramethylbutyl)-phenol] is stirred for 8 hours at 160˚C with 0.5 ml of N-methyl-2-pyrrolidone and 0.072 g (1.105 mmol) of sodium azide. After the reaction has taken place, according to HPLC analysis the reaction mass contains 24 % by weight of 2,2-methylenebis[6-(2H-benzotriazol-2-yl)-4-(1,1,3,3-tetramethylbutyl)-phenol (compound 114, Table 1, corresponding to Tinuvin 360 (RTM), Ciba Spezialitätenchemie AG).

Example 19: Preparation of a mixture of compounds 115, 116, 107 (Table 1).

43.26 g (115 mmol) of 3-(1,1-dimethylethyl)-4-hydroxy-5-[(2-chlorophenyl)-azo]-benzene-propanoic acid methyl ester [compound 209 (Table 2), prepared according to Example 9a] is stirred for 30 minutes at 150˚C and then for 13 hours at from 160 to 170˚C with 36.0 g (120 mmol) of polyethylene glycol 300, 9.75 g (150 mmol) of sodium azide, 0.5 ml of triethylamine and 85.6 mg (1.15 mmol) of CuBr, during which methanol is continuously distilled off. The mixture is then evacuated to 200 mbar and maintained for a further 2 hours at from 160 to 170˚C. After the reaction has taken place, according to HPLC analysis the liquid component of the reaction mass contains 56.7 % of compound 115 (Table 1), 30.4 % of compound 116 (Table 1) and 1.5 % of compound 107 (Table 1).

Example 20: Preparation of compound 117 (Table 1).

25 ml of dimethylformamide, 5 g (38.4 mmol) of isooctanol (isomeric mixture) and 1.35 g (20.8 mmol) of sodium azide are added to 6.12 g (16 mmol) of 3-(1,1-dimethylethyl)-4-hydroxy-5-[(2-nitrophenyl)-azo]-benzenepropanoic acid methyl ester [compound 213, Table 2, prepared analogously to Example 9a] and the mixture is stirred at 150˚C. Methanol/DMF is distilled off at the outset at normal pressure and after 5 hours at slightly reduced pressure. After 6 hours, a further 2.5 g (19.2 mmol) of isooctanol is added and the temperature is tained at 150˚C for a further 5.5 hours. After the reaction has taken place, according to GC analysis the liquid components of the reaction mass contain up to 35 % of compound 117 (Table 1).

Table 1:

| Compound | Structural formula |
|---|---|
| 101 (reference compound) | 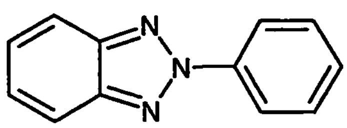 |
| 102 (reference compound) | 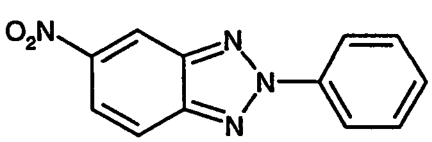 |

(continued)

| Compound | Structural formula |
|---|---|
| 103 | 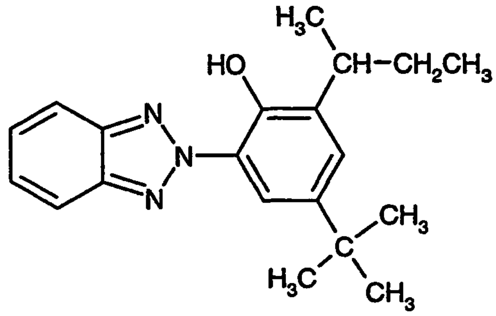 |
| 104 | 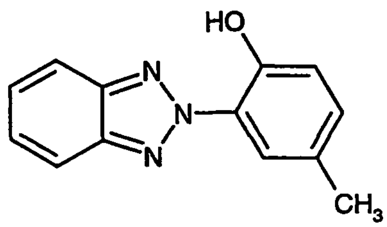 |
| 105 | 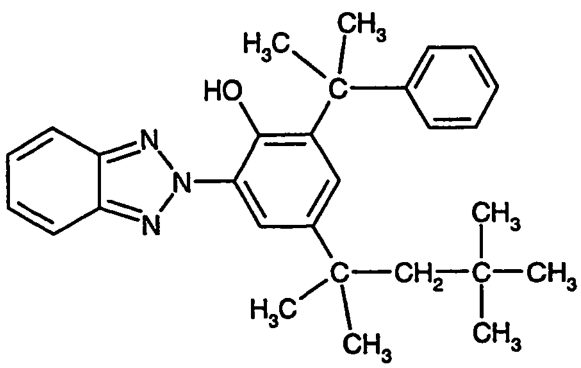 |
| 106 | 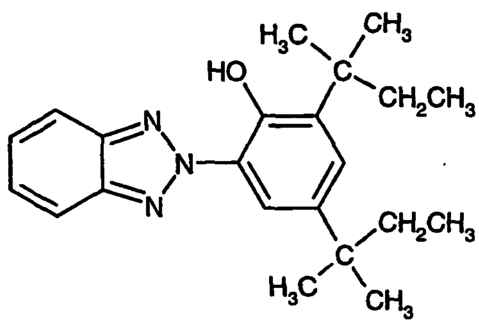 |
| 107 | 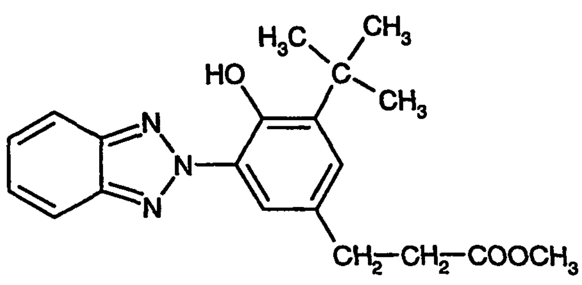 |

(continued)

| Compound | Structural formula |
|---|---|
| 108 | 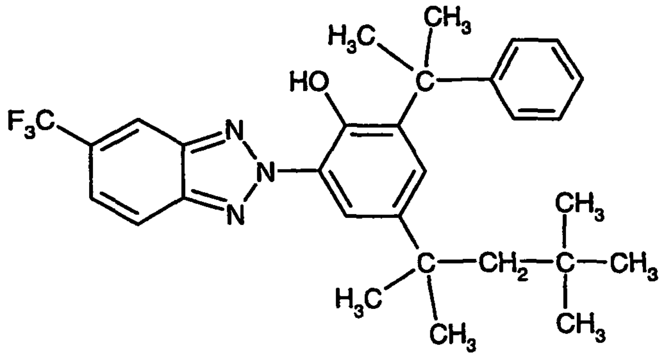 |
| 109 | 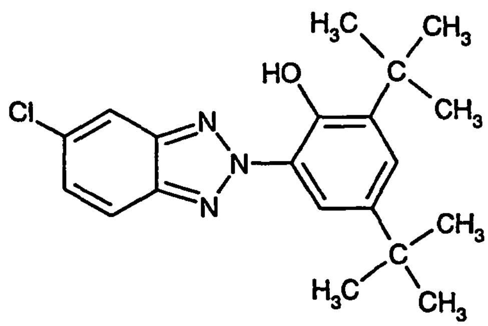 |
| 110 | 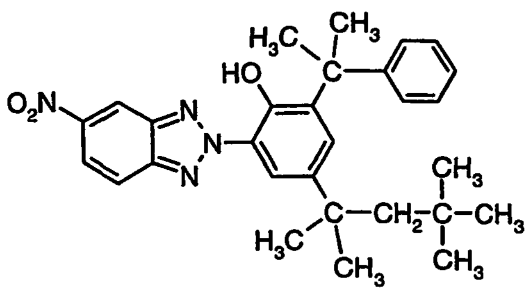 |
| 111 | 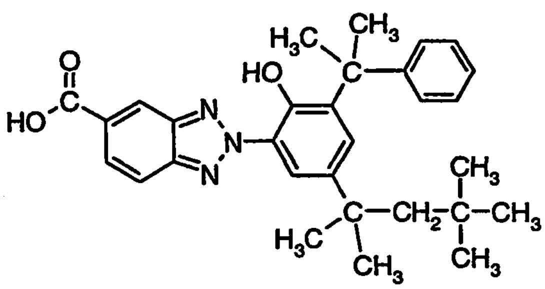 |

(continued)

| Compound | Structural formula |
|---|---|
| 112 | 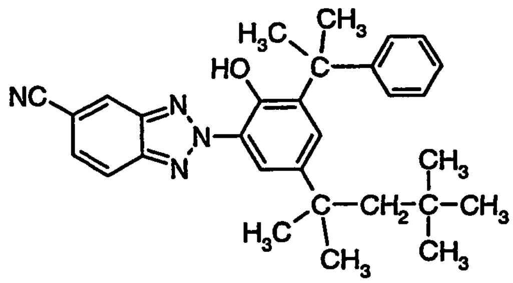 |
| 113 | 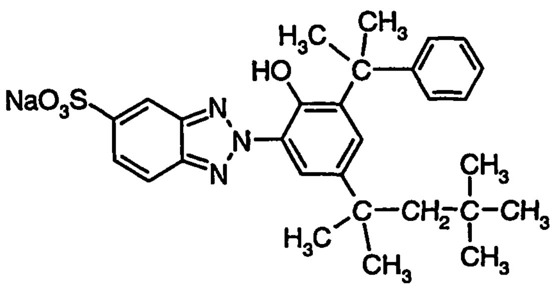 |
| 114 | 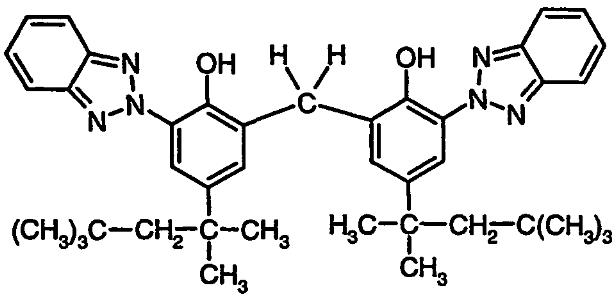 |
| 115 | 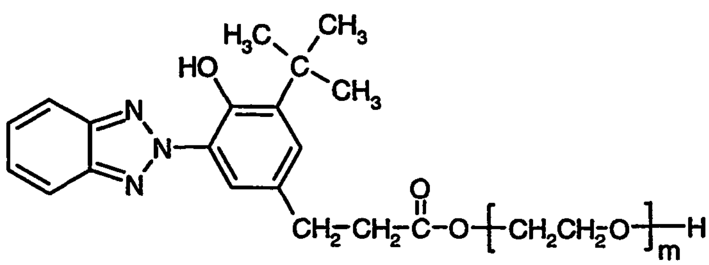 |
| 116 | 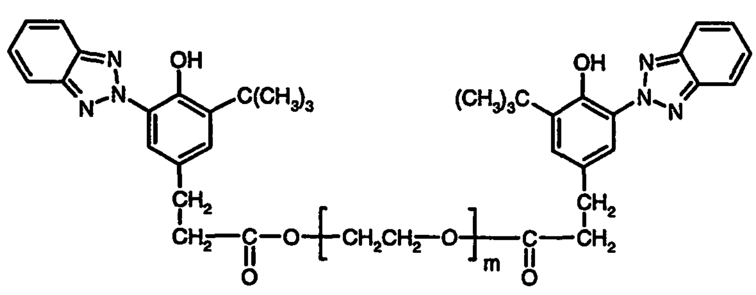 |

(continued)

| Compound | Structural formula |
|---|---|
| 117 | 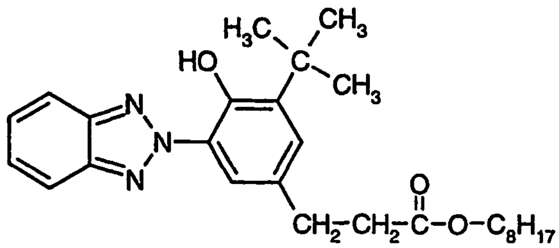 |

Table 2:

| Compound | Structural formula |
|---|---|
| 201<br>(reference compound) | 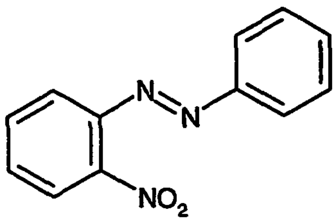 |
| 202<br>(reference compound) | 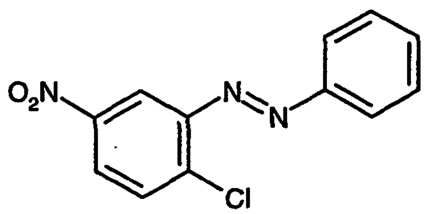 |
| 203 | 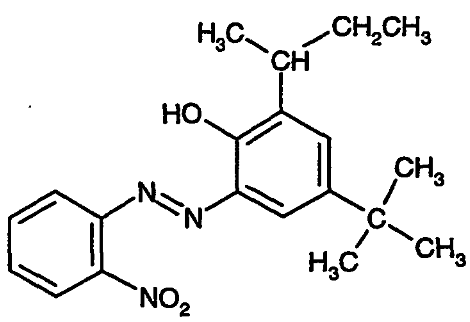 |
| 204 | 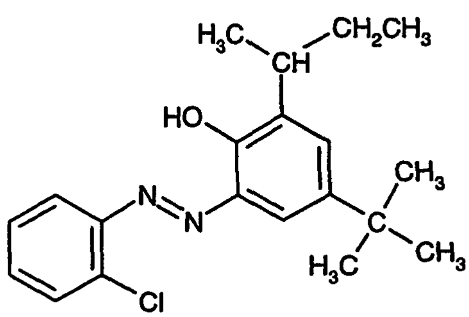 |

(continued)

| Compound | Structural formula |
|---|---|
| 205 | 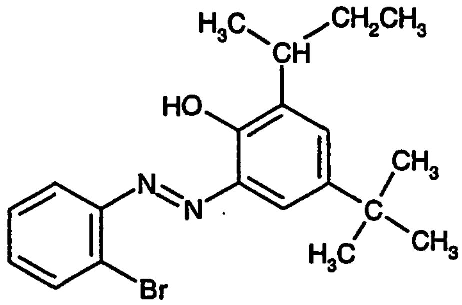 |
| 206 | 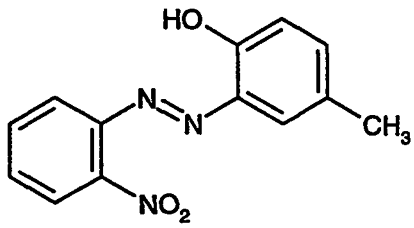 |
| 207 | 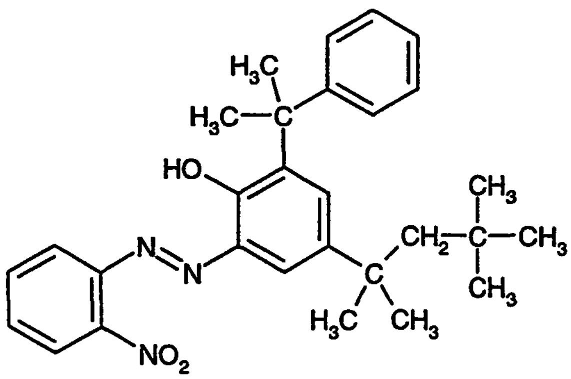 |
| 208 | 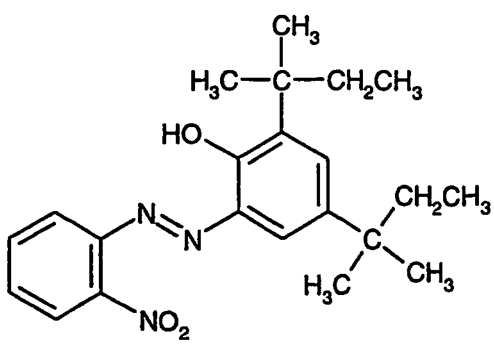 |

(continued)

| Compound | Structural formula |
|---|---|
| 209 | 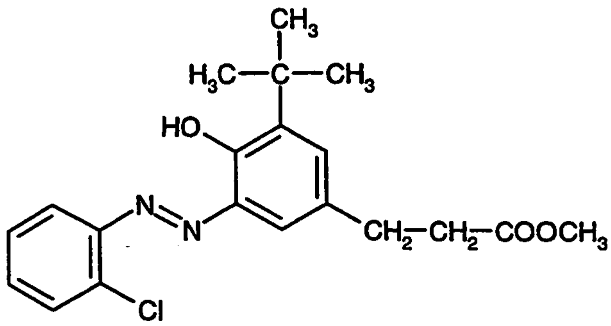 |
| 210 | 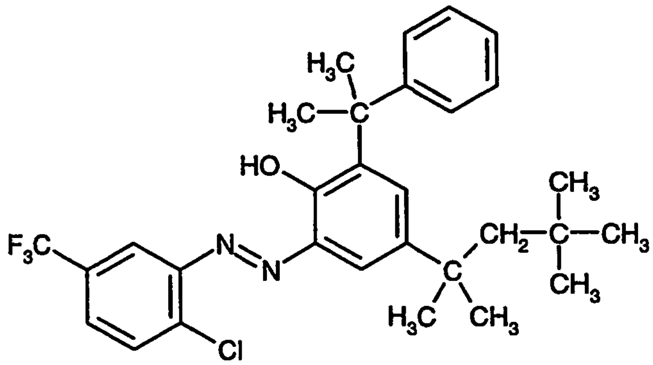 |
| 211 | 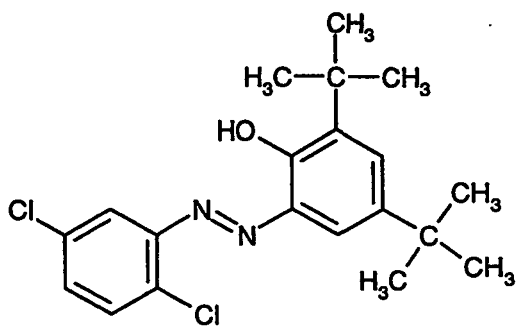 |
| 212 | 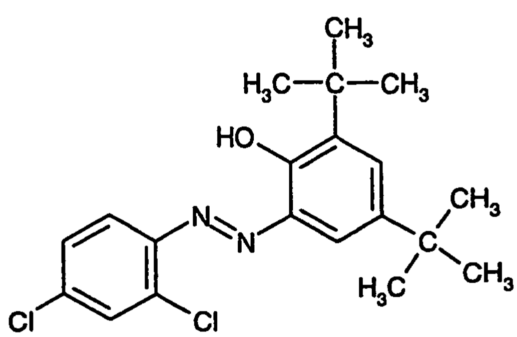 |

(continued)

| Compound | Structural formula |
|---|---|
| 213 | 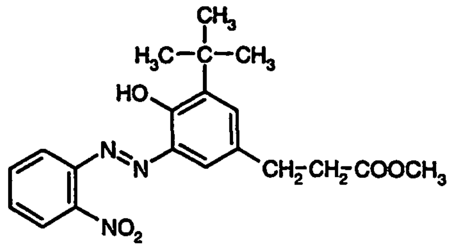 |
| 214 | 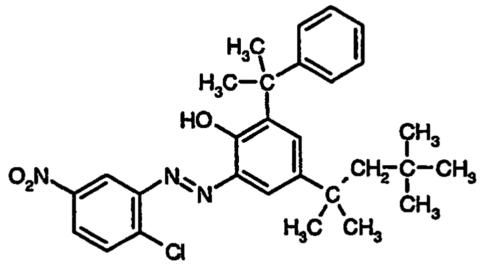 |
| 215 | 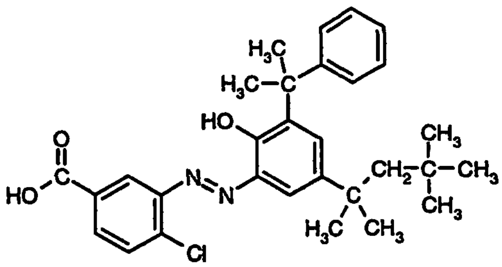 |
| 216 | 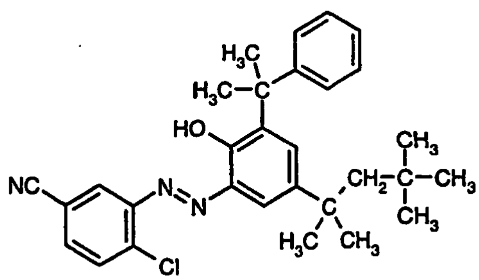 |
| 217 | 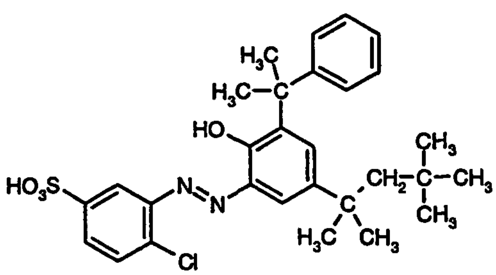 |

(continued)

| Compound | Structural formula |
|---|---|
| 218 | 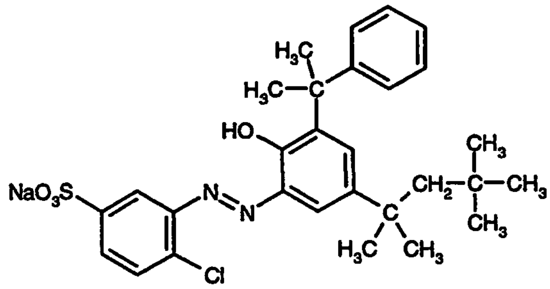 |
| 219 | 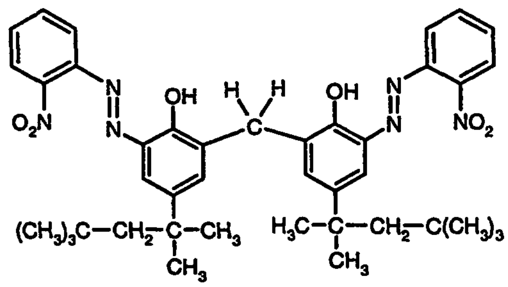 |

## Claims

**1.** A process for the preparation of a compound of formula I

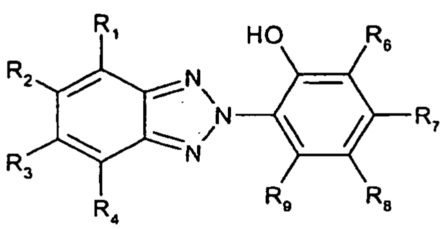

(I)

wherein

$R_1$, $R_2$, $R_3$, $R_4$, $R_6$, $R_7$, $R_8$ and $R_9$ are each independently of the others hydrogen, halogen, $-SO_3H$, $-SO_3^- M_a^+$, hydroxy, carboxy, cyano, nitro, $C_1$-$C_{25}$alkyl, $C_1$-$C_{25}$alkyl substituted by halogen, hydroxy, carboxy, cyano, $C_1$-$C_{18}$alkoxycarbonyl, $C_1$-$C_4$alkoxy or by amino; $C_2$-$C_{24}$alkenyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$cydoalkyl; $C_1$-$C_{18}$alkoxy, $C_1$-$C_{18}$alkylthio, $C_1$-$C_{18}$alkylsulfonyl, unsubstituted or $C_1$-$C_4$alkyl-substituted phenylsulfonyl, unsubstituted or $C_1$-$C_4$alkyl-substituted phenylthio; amino, $C_1$-$C_4$alkylamino, di($C_1$-$C_4$alkyl)amino, $C_1$-$C_{25}$alkanoyl, $C_1$-$C_{25}$alkoxycarbonyl, $C_1$-$C_{25}$alkanoyloxy, $C_1$-$C_{25}$alkanoylamino, $C_3$-$C_{25}$alkyl interrupted by oxygen, sulfur or by

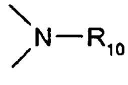 ;

$C_3$-$C_{25}$alkoxy interrupted by oxygen, sulfur or by

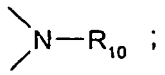

$C_3$-$C_{25}$alkanoyloxy interrupted by oxygen, sulfur or by

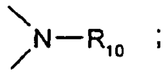

$C_3$-$C_{25}$alkoxycarbonyl interrupted by oxygen, sulfur or by

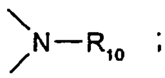

$C_6$-$C_9$cycloalkoxycarbonyl, $C_6$-$C_9$cycloalkylcarbonyloxy, unsubstituted or $C_1$-$C_{12}$alkyl-substituted benzoyloxy; $-(CH_2)_p-COR_{11}$ or $-(CH_2)_qOH$, or, further, the radicals $R_6$ and $R_7$ or the radicals $R_7$ and $R_8$ or the radicals $R_8$ and $R_9$, together with the carbon atoms to which they are bonded, form a benzo ring, $R_3$ in addition is a radical of formula II

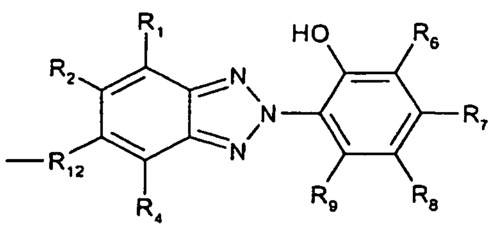

(II),

and $R_6$ in addition is a radical of formula III

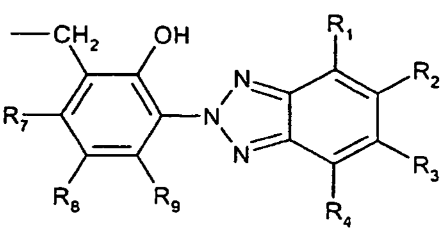

(III),

$R_{10}$ is hydrogen or $C_1$-$C_8$alkyl,
$R_{11}$ is hydroxy,

$$\left[-O^- \frac{1}{r} M_b^{r+}\right] ,$$

$C_1$-$C_{18}$alkoxy,

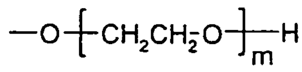 , 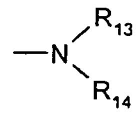

or a radical of formula IV

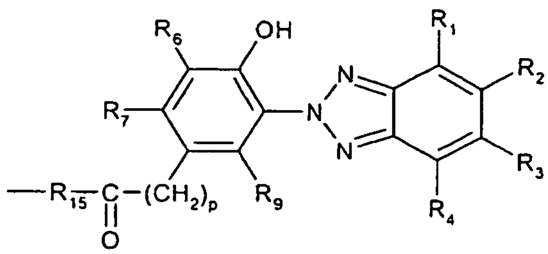 (IV),

$R_{12}$ is -SO-, $-SO_2-$, $-SO-R_{16}-SO-$, $-SO_2-R_{16}-SO_2-$,

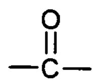 , 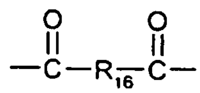 ,

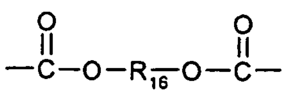 , 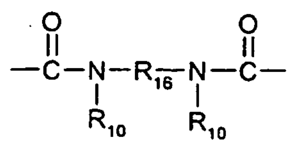 or 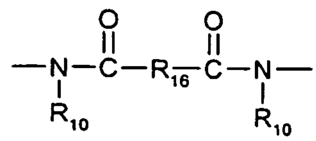 ,

$R_{13}$ and $R_{14}$ are each independently of the other hydrogen or $C_1$-$C_{18}$alkyl,
$R_{15}$ is

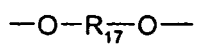 ,

$R_{16}$ is $C_2$-$C_{12}$alkylene, $C_5$-$C_{12}$cycloalkylene, or $C_8$-$C_{12}$alkylene interrupted or terminated by cyclohexylene;
$R_{17}$ is $C_2$-$C_{12}$alkylene, or $C_4$-$C_{12}$alkylene interrupted by oxygen, sulfur or by

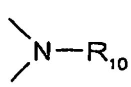 ,

$M_a$ is a monovalent metal cation,
$M_b$ is an r-valent metal cation,
m is an integer from 1 to 20,
p is 0, 1 or 2,
q is 1, 2, 3, 4, 5 or 6, and
r is 1, 2 or 3,

which process comprises reacting a compound of formula V

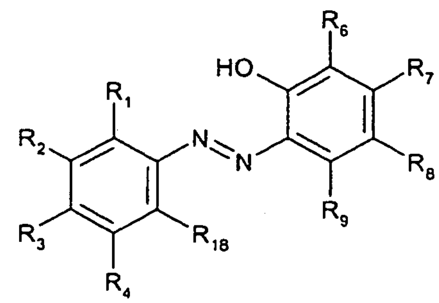

(V)

wherein

$R_1$, $R_2$, $R_3$, $R_4$, $R_6$, $R_7$, $R_8$ and $R_9$ are as defined hereinbefore, $R_3$ in addition being a radical of formula VI

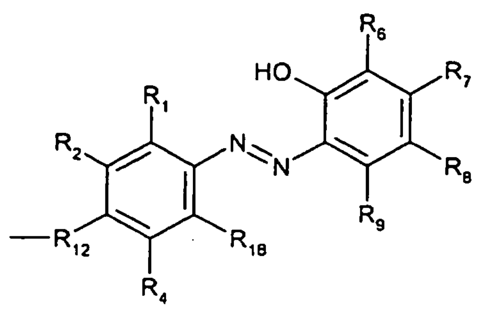

(VI),

$R_6$ in addition being a radical of formula VII

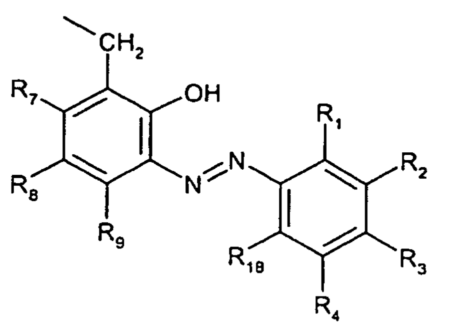

(VII)

and $R_{11}$ in addition being a radical of formula VIII

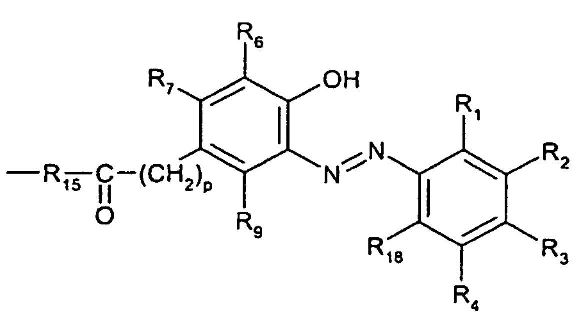

(VIII),

$R_{18}$ is halogen, nitro,

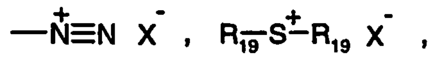

$-SO_3H$,

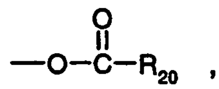

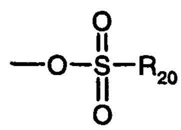

or $C_1$-$C_{18}$alkoxy,
$R_{19}$ is $C_1$-$C_{18}$alkyl, unsubstituted or $C_1$-$C_4$alkyl-substituted phenyl; or unsubstituted or $C_1$-$C_4$alkyl-substituted $C_5$-$C_8$cycloalkyl,
$R_{20}$ is $C_1$-$C_{18}$alkyl, unsubstituted or $C_1$-$C_4$alkyl-, halo- or nitro-substituted phenyl; unsubstituted or $C_1$-$C_4$alkyl-substituted $C_5$-$C_8$cycloalkyl; or fluorine-substituted $C_1$-$C_{18}$alkyl, and
$X^-$ is chloride, bromide, iodide, hydroxide, nitrate or nitrite,

with an azide compound of formula IX

$$M^{n+}\ (N_3^-)_n \qquad (IX)$$

wherein

M is an n-valent metal cation,

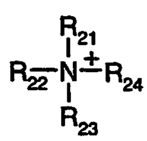

or $P^+(R_{25})_4$,
$R_{21}$, $R_{22}$, $R_{23}$ and $R_{24}$ are each independently of the others hydrogen or $C_1$-$C_{18}$alkyl,
$R_{25}$ is $C_1$-$C_{18}$alkyl, and
n is 1, 2 or 3.

**2.** A process according to claim 1, wherein $R_{11}$ is hydroxy,

$$\left[-O^- \ \frac{1}{r} M_b^{r+}\right] ,$$

$C_1$-$C_{18}$alkoxy,

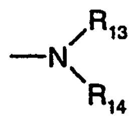

or a radical of formula IV;
$R_{13}$ and $R_{14}$ are each independently of the other hydrogen or $C_1$-$C_{18}$alkyl,
$M_b$ is an r-valent metal cation, and
r is 1, 2 or 3.

**3.** A process according to claim 1, wherein
$R_1$, $R_2$, $R_3$, $R_4$, $R_6$, $R_7$, $R_8$ and $R_9$ are each independently of the others hydrogen, halogen, - $SO_3H$, $-SO_3^-$ $M_a^+$, hydroxy, carboxy, cyano, nitro, $C_1$-$C_{18}$alkyl, fluorine- or $C_1$-$C_4$alkoxy-substituted $C_1$-$C_{18}$alkyl; $C_2$-$C_{18}$alkenyl, $C_7$-$C_9$phenylalkyl, unsubstituted or $C_1$-$C_4$alkyl-substituted phenyl; $C_5$-$C_8$cycloalkyl, $C_1$-$C_{18}$alkoxy, $C_1$-$C_{18}$alkylthio, $C_1$-$C_{18}$alkylsulfonyl, phenylsulfonyl, phenylthio, $C_1$-$C_4$alkylamino, di($C_1$-$C_4$alkyl)amino, $C_1$-$C_{18}$alkanoyl, $C_1$-$C_{18}$alkoxycarbonyl, $C_1$-$C_{18}$alkanoyloxy, $C_1$-$C_{18}$alkanoylamino, $C_3$-$C_{18}$alkyl interrupted by oxygen, sulfur or by

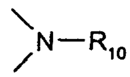 ;

$C_3$-$C_{18}$alkoxy interrupted by oxygen, sulfur or by

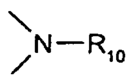 ;

$C_3$-$C_{18}$alkanoyloxy interrupted by oxygen, sulfur or by

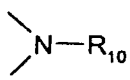 ;

$C_3$-$C_{18}$alkoxycarbonyl interrupted by oxygen, sulfur or by

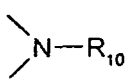 ;

$C_6$-$C_9$cycloalkoxycarbonyl, $C_6$-$C_9$cycloalkylcarbonyloxy, unsubstituted or $C_1$-$C_4$alkyl-substituted benzoyloxy; $-(CH_2)_p$-$COR_{11}$ or $-(CH_2)_q$OH, or, further, the radicals $R_6$ and $R_7$ or the radicals $R_7$ and $R_8$ or the radicals $R_8$ and $R_9$, together with the carbon atoms to which they are bonded, form a benzo ring, $R_3$ in addition is a radical of formula II and $R_6$ in addition is a radical of formula III,
$R_{10}$ is hydrogen or $C_1$-$C_6$alkyl,
$R_{11}$ is hydroxy,

$$\left[-O^{-}\ \frac{1}{r}M_b^{r+}\right] ,$$

$C_1$-$C_{12}$alkoxy,

$-O-[CH_2CH_2-O]_m-H$ , 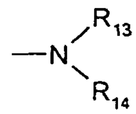

or a radical of formula IV,
$R_{12}$ is $-SO_2-$, $-SO_2-R_{16}-SO_2-$,

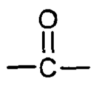 , 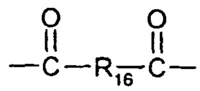 , 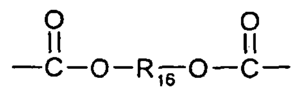

or

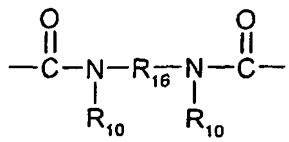 ,

$R_{13}$ and $R_{14}$ are each independently of the other hydrogen or $C_1$-$C_8$alkyl,
$R_{15}$ is

$-O-R_{17}-O-$ ,

$R_{16}$ is $C_2$-$C_{12}$alkylene or $C_5$-$C_{12}$cycloalkylene,
$R_{17}$ is $C_2$-$C_8$alkylene, or $C_4$-$C_{12}$alkylene interrupted by oxygen or by sulfur,
$R_{18}$ is halogen, nitro,

$-\overset{+}{N}\equiv N\ X^-$ , $R_{19}-\overset{+}{S}-R_{19}\ X^-$ , 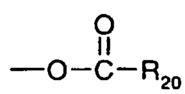 , 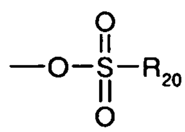

or $C_1$-$C_{12}$alkoxy,
$R_{19}$ is $C_1$-$C_{18}$alkyl, phenyl or $C_5$-$C_8$cycloalkyl,
$R_{20}$ is $C_1$-$C_{18}$alkyl, unsubstituted or $C_1$-$C_4$alkyl-, fluorine-, chlorine- or nitro-substituted phenyl; $C_5$-$C_8$cycloalkyl, or fluorine-substituted $C_1$-$C_{12}$alkyl,
$R_{21}$, $R_{22}$, $R_{23}$ and $R_{24}$ are each independently of the others hydrogen or $C_1$-$C_{12}$alkyl,
$R_{25}$ is $C_1$-$C_{12}$alkyl,
M is lithium, sodium, potassium, calcium,

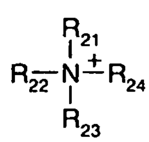

or $P^+(R_{25})_4$,
$M_a$ is sodium or potassium,
$M_b$ is sodium, potassium or calcium,

$X^-$ is chloride or bromide,
m is an integer from 1 to 15,
n is 1 or 2,
p is 0, 1 or 2,
q is 1, 2 or 3, and
r is 1 or 2.

**4.** A process according to claim 1, wherein
$R_1$ is hydrogen, chlorine, carboxy, nitro, $C_1$-$C_4$alkyl, trifluoromethyl or $C_1$-$C_4$alkoxy,
$R_2$ is hydrogen, chlorine, $-SO_3H$, $-SO_3^-M_a^+$, carboxy, cyano, nitro, $C_1$-$C_4$alkyl, trifluoromethyl, $C_1$-$C_4$alkoxy, benzyl, phenyl, cyclohexyl, $C_1$-$C_4$alkylsulfonyl, phenylsulfonyl, $C_1$-$C_8$alkanoyl, $C_1$-$C_8$alkoxycarbonyl, $C_1$-$C_8$alkanoyloxy, $C_3$-$C_8$alkyl interrupted by oxygen or by sulfur; $C_3$-$C_8$alkoxy interrupted by oxygen or by sulfur; $C_3$-$C_8$alkanoyloxy interrupted by oxygen or by sulfur; $C_3$-$C_8$alkoxycarbonyl interrupted by oxygen or by sulfur; cyclohexyloxycarbonyl, cyclohexylcarbonyloxy, or benzoyloxy,
$R_3$ is hydrogen or a radical of formula II,
$R_4$ is hydrogen, chlorine, carboxy, nitro, $C_1$-$C_4$alkyl, trifluoromethyl or $C_1$-$C_4$alkoxy,
$R_6$ is hydrogen, $C_1$-$C_{12}$alkyl, $C_7$-$C_9$phenylalkyl, phenyl, cyclohexyl, $C_1$-$C_{12}$alkoxy, $C_3$-$C_{12}$alkyl interrupted by oxygen or by sulfur; $C_3$-$C_{12}$alkoxy interrupted by oxygen or by sulfur; or a radical of formula III,
$R_7$ is hydrogen, chlorine, $C_1$-$C_4$alkyl or $C_1$-$C_4$alkoxy,
$R_8$ is hydrogen, $C_1$-$C_{12}$alkyl, $C_7$-$C_9$phenylalkyl, phenyl, cyclohexyl, $C_1$-$C_{12}$alkoxy, $C_3$-$C_{12}$alkyl interrupted by oxygen or by sulfur; $C_3$-$C_{12}$alkoxy interrupted by oxygen or by sulfur; or $-(CH_2)_p-COR_{11}$,
$R_9$ is hydrogen, chlorine, $C_1$-$C_4$alkyl or $C_1$-$C_4$alkoxy,
$R_{11}$ is hydroxy, $C_1$-$C_8$alkoxy,

$$-O-[CH_2CH_2O]_m-H$$

or a radical of formula IV,
$R_{12}$ is $-SO_2-$,

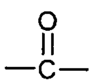 , 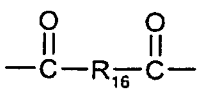 or 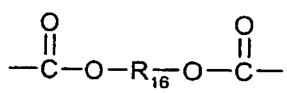 ,

$R_{15}$ is

$$-O-R_{17}-O- ,$$

$R_{16}$ is $C_2$-$C_{18}$alkylene or $C_5$-$C_8$cycloalkylene,
$R_{17}$ is $C_2$-$C_8$alkylene, or $C_4$-$C_{12}$alkylene interrupted by oxygen,
$R_{18}$ is chlorine, bromine, iodine, nitro,

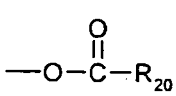 or 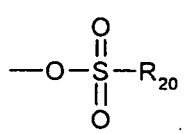 ,

$R_{20}$ is $C_1$-$C_8$alkyl, unsubstituted or fluorine-, chlorine- or nitro-substituted phenyl; or fluorine-substituted $C_1$-$C_4$alkyl,
M is lithium, sodium, potassium or calcium,
$M_a^+$ is sodium or potassium,
m is an integer from 1 to 15,
n is 1 or 2, and

p is 1 or 2.

**5.** A process according to claim 1, wherein $R_1$, $R_4$, $R_7$ and $R_9$ are hydrogen.

**6.** A process according to claim 1, wherein $R_{18}$ is nitro, chlorine or bromine.

**7.** A process according to claim 1, wherein
$R_1$ is hydrogen or $C_1$-$C_4$alkyl,
$R_2$ is hydrogen, chlorine, $-SO_3H$, $-SO_3^- M_a^+$, carboxy, cyano, nitro, $C_1$-$C_4$alkyl, trifluoromethyl, $C_1$-$C_4$alkoxy, $C_1$-$C_4$alkanoyl, $C_1$-$C_4$alkoxycarbonyl, $C_3$-$C_8$alkyl interrupted by oxygen; or $C_3$-$C_8$alkoxy interrupted by oxygen,
$R_3$ is hydrogen,
$R_4$ is hydrogen or $C_1$-$C_4$alkyl,
$R_6$ is hydrogen, $C_1$-$C_8$alkyl, $C_7$-$C_9$phenylalkyl, phenyl, cyclohexyl or a radical of formula III,
$R_7$ is hydrogen or $C_1$-$C_4$alkyl,
$R_8$ is hydrogen, $C_1$-$C_{12}$alkyl, $C_7$-$C_9$phenylalkyl, phenyl, cyclohexyl or $-(CH_2)_p-COR_{11}$,
$R_9$ is hydrogen or $C_1$-$C_4$alkyl,
$R_{11}$ is hydroxy, $C_1$-$C_8$alkoxy,

$$-O-\left[CH_2CH_2O\right]_m-H$$

or a radical of formula IV,
$R_{15}$ is

$$-O-R_{17}-O- \quad ,$$

$R_{17}$ is $C_4$-$C_{12}$alkylene interrupted by oxygen,
$R_{18}$ is chlorine, bromine, nitro,

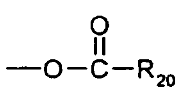 or 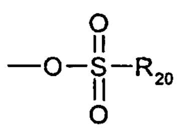 ,

$R_{20}$ is $C_1$-$C_4$alkyl, unsubstituted or fluorine-, chlorine- or nitro-substituted phenyl; or trifluoromethyl,
M is lithium, sodium or potassium,
$M_a^+$ is sodium or potassium,
m is an integer from 1 to 10,
n is 1, and
p is 2.

**8.** A process according to claim 1, wherein
$R_1$ is hydrogen,
$R_2$ is hydrogen, chlorine, $-SO_3H$, $-SO_3^- M_a^+$, carboxy, cyano, nitro or trifluoromethyl,
$R_3$ is hydrogen,
$R_4$ is hydrogen,
$R_6$ is hydrogen, $C_1$-$C_5$alkyl, $\alpha,\alpha$-dimethylbenzyl or a radical of formula III,
$R_7$ is hydrogen,
$R_8$ is hydrogen, $C_1$-$C_8$alkyl or $-(CH_2)_p-COR_{11}$,
$R_9$ is hydrogen,
$R_{11}$ is $C_1$-$C_8$alkoxy,

$$-O-\left[CH_2CH_2-O\right]_m-H$$

or a radical of formula IV,
$R_{15}$ is

$$-O-R_{17}-O- \quad ,$$

$R_{17}$ is $C_4$-$C_{12}$alkylene interrupted by oxygen,
$R_{18}$ is nitro, chlorine or bromine,
M is lithium or sodium,
$M_a^+$ is sodium or potassium,
m is an integer from 1 to 10,
n is 1, and
p is 2.

**9.** A process according to claim 1, wherein the reaction is carried out in a solvent.

**10.** A process according to claim 1, wherein the molar ratio of the amount of compound of formula V to the amount of azide compound of formula IX is from 1 : 1 to 1 : 3.

**11.** A process according to claim 1, wherein the reaction is carried out in the presence of a catalyst.

**Patentansprüche**

**1.** Verfahren zur Herstellung von Verbindungen der Formel I

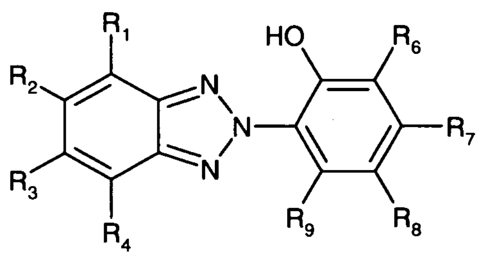

(I)

worin

$R_1$, $R_2$, $R_3$, $R_4$, $R_6$, $R_7$, $R_8$ und $R_9$ unabhängig voneinander Wasserstoff, Halogen, $-SO_3H$, $-SO_3^- M_a^+$, Hydroxy, Carboxy, Cyano, Nitro, $C_1$-$C_{25}$-Alkyl, durch Halogen, Hydroxy, Carboxy, Cyano, $C_1$-$C_{18}$-Alkoxycarbonyl, $C_1$-$C_4$-Alkoxy oder Amino substituiertes $C_1$-$C_{25}$-Alkyl; $C_2$-$C_{24}$-Alkenyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, $C_1$-$C_{18}$-Alkylsulfonyl, unsubstituiertes oder durch $C_1$-$C_4$-Alkyl substituiertes Phenylsulfonyl, unsubstituiertes oder durch $C_1$-$C_4$-Alkyl substituiertes Phenylthio, Amino, $C_1$-$C_4$-Alkylamino, Di-($C_1$-$C_4$-alkyl)amino, $C_1$-$C_{25}$-Alkanoyl, $C_1$-$C_{25}$-Alkoxycarbonyl, $C_1$-$C_{25}$-Alkanoyloxy, $C_1$-$C_{25}$-Alkanoylamino, durch Sauerstoff, Schwefel oder

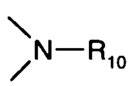

unterbrochenes $C_3$-$C_{25}$-Alkyl; durch Sauerstoff, Schwefel oder

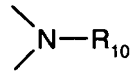

unterbrochenes $C_3$-$C_{25}$-Alkoxy; durch Sauerstoff, Schwefel oder

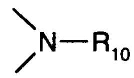

unterbrochenes $C_3$-$C_{25}$-Alkanoyloxy; durch Sauerstoff, Schwefel oder

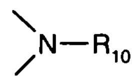

unterbrochenes $C_3$-$C_{25}$-Alkoxycarbonyl; $C_6$-$C_9$-Cycloalkoxycarbonyl, $C_6$-$C_9$-Cycloalkylcarbonyloxy, unsubstituiertes oder durch $C_1$-$C_{12}$-Alkyl substituiertes Benzoyloxy; $-(CH_2)_p-COR_{11}$ oder $-(CH_2)_qOH$ darstellen, oder ferner die Reste $R_6$ und $R_7$ oder die Reste $R_7$ und $R_8$ oder die Reste $R_8$ und $R_9$ zusammen mit den Kohlenstoffatomen, an die sie gebunden sind, einen Benzoring bilden, $R_3$ zusätzlich einen Rest der Formel II

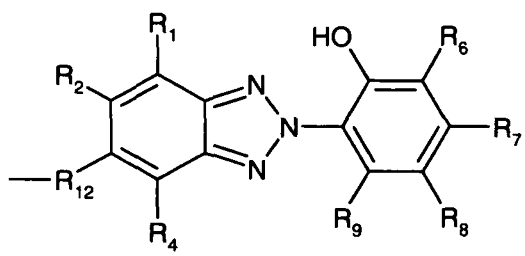

(II)

bedeutet, $R_6$ zusätzlich einen Rest der Formel III

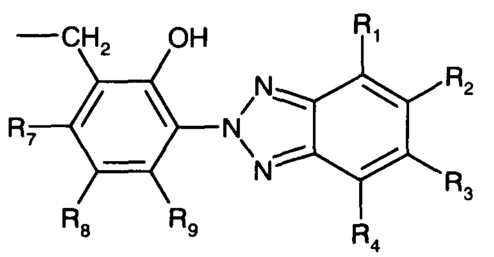

(III)

darstellt,
$R_{10}$ Wasserstoff oder $C_1$-$C_8$-Alkyl ist,
$R_{11}$ Hydroxy,

$$\left[-O^{-}\ \frac{1}{r}M_b^{r+}\right]\ ,$$

$C_1$-$C_{18}$-Alkoxy,

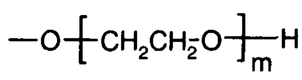 , 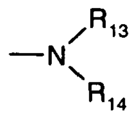

oder einen Rest der Formel IV

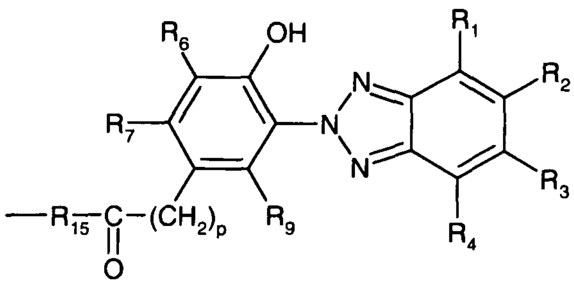 (IV)

bedeutet,
$R_{12}$ -SO-, -$SO_2$-, -SO-$R_{16}$-SO-, -$SO_2$-$R_{16}$-$SO_2$-,

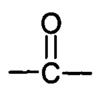 , 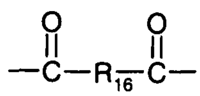 ,

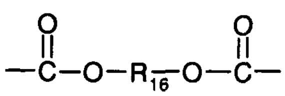 , 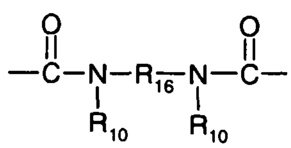 oder 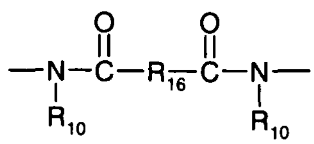

darstellt,
$R_{13}$ und $R_{14}$ unabhängig voneinander Wasserstoff oder $C_1$-$C_{18}$-Alkyl bedeuten,
$R_{15}$

$$-O-R_{17}-O-$$

darstellt,
$R_{16}$ $C_2$-$C_{12}$-Alkylen, $C_5$-$C_{12}$-Cycloalkylen, oder mit Cyclohexylen unterbrochenes oder beendetes $C_8$-$C_{12}$-Alkylen bedeutet;
$R_{17}$ $C_2$-$C_{12}$-Alkylen, durch Sauerstoff, Schwefel oder

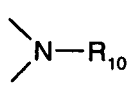

unterbrochenes $C_4$-$C_{12}$-Alkylen darstellt,
$M_a$ ein einwertiges Metallkation ist,

$M_b$ ein r-wertiges Metallkation bedeutet,
m eine ganze Zahl von 1 bis 20 darstellt,
p 0, 1 oder 2 ist,
q 1, 2, 3, 4, 5 oder 6 darstellt, und
r 1, 2 oder 3 ist,

**dadurch gekennzeichnet, dass** eine Verbindung der Formel V

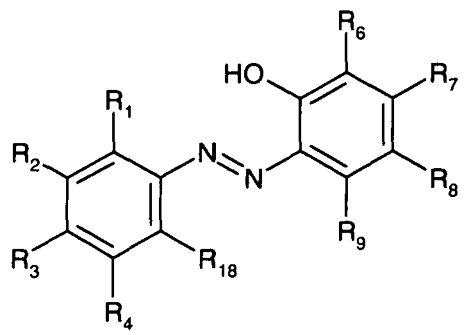
(V)

worin

$R_1$, $R_2$, $R_3$, $R_4$, $R_6$, $R_7$, $R_8$ und $R_9$ wie oben angegeben, definiert sind, $R_3$ zusätzlich einen Rest der Formel VI

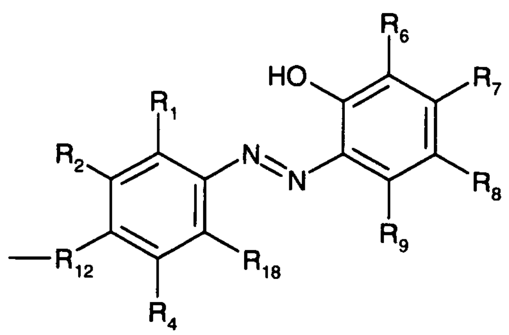
(VI)

bedeutet, $R_6$ zusätzlich einen Rest der Formel VII

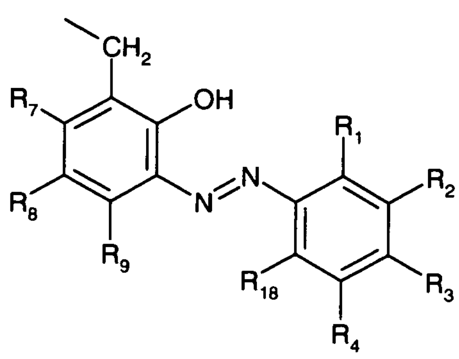
(VII)

darstellt, und $R_{11}$ zusätzlich einen Rest der Formel VIII

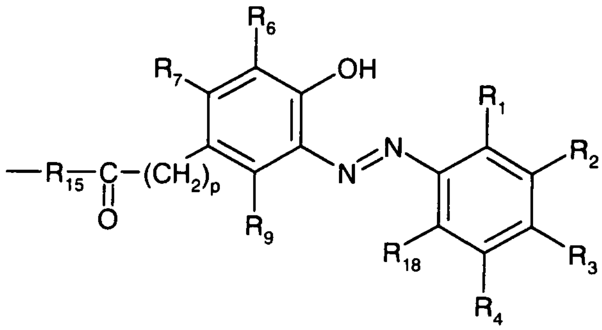

(VIII)

bedeutet,

$R_{18}$ Halogen, Nitro,

$$-\overset{+}{N}{\equiv}N\ X^{-}\ ,\quad R_{19}-\overset{+}{S}-R_{19}\ X^{-}\ ,$$

$-SO_3H$,

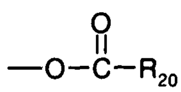

,

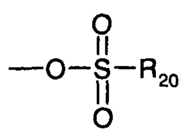

oder $C_1$-$C_{18}$-Alkoxy darstellt,

$R_{19}$ $C_1$-$C_{18}$-Alkyl, unsubstituiertes oder durch $C_1$-$C_4$-Alkyl substituiertes Phenyl; unsubstituiertes oder durch $C_1$-$C_4$-Alkyl substituiertes $C_5$-$C_8$-Cycloalkyl bedeutet,

$R_{20}$ $C_1$-$C_{18}$-Alkyl, unsubstituiertes oder durch $C_1$-$C_4$-Alkyl, Halogen oder Nitro substituiertes Phenyl; unsubstituiertes oder durch $C_1$-$C_4$-Alkyl substituiertes $C_5$-$C_8$-Cycloalkyl; durch Fluor substituiertes $C_1$-$C_{18}$-Alkyl darstellt, und

$X^-$ Chlorid, Bromid, Jodid, Hydroxid, Nitrat oder Nitrit bedeutet,

mit einer Azid-Verbindung der Formel IX

$$M^{n+}\ (N_3^{-})_n \qquad \text{(IX)}$$

worin

M ein n-wertiges Metallkation,

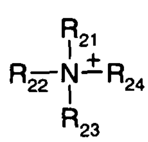

oder $P^+(R_{25})_4$ darstellt,
$R_{21}$, $R_{22}$, $R_{23}$ und $R_{24}$ unabhängig voneinander Wasserstoff oder $C_1$-$C_{18}$-Alkyl bedeuten,
$R_{25}$ $C_1$-$C_{18}$-Alkyl darstellt, und
n 1, 2 oder 3 ist, umgesetzt wird.

**2.** Verfahren gemäss Anspruch 1, worin $R_{11}$ Hydroxy,

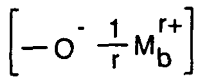 ,

$C_1$-$C_{18}$-Alkoxy,

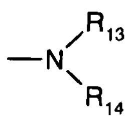

oder einen Rest der Formel IV bedeutet;
$R_{13}$ und $R_{14}$ unabhängig voneinander Wasserstoff oder $C_1$-$C_{18}$-Alkyl darstellen,
$M_b$ ein r-wertiges Metallkation bedeutet, und
r 1, 2 oder 3 ist.

**3.** Verfahren gemäss Anspruch 1, worin
$R_1$, $R_2$, $R_3$, $R_4$, $R_6$, $R_7$, $R_8$ und $R_9$ unabhängig voneinander Wasserstoff, Halogen, $-SO_3H$, $-SO_3^-$ $M_a^+$, Hydroxy, Carboxy, Cyano, Nitro, $C_1$-$C_{18}$-Alkyl, durch Fluor oder $C_1$-$C_4$-Alkoxy substituiertes $C_1$-$C_{18}$-Alkyl; $C_2$-$C_{18}$-Alkenyl, $C_7$-$C_9$-Phenylalkyl, unsubstituiertes oder durch $C_1$-$C_4$-Alkyl substituiertes Phenyl, $C_5$-$C_8$-Cycloalkyl, $C_1$-$C_{18}$-Alkoxy, $C_1$-$C_{18}$-Alkylthio, $C_1$-$C_{18}$-Alkylsulfonyl, Phenylsulfonyl, Phenylthio, $C_1$-$C_4$-Alkylamino, Di-($C_1$-$C_4$-alkyl)amino, $C_1$-$C_{18}$-Alkanoyl, $C_1$-$C_{18}$-Alkoxycarbonyl, $C_1$-$C_{18}$-Alkanoyloxy, $C_1$-$C_{18}$-Alkanoylamino, durch Sauerstoff, Schwefel oder

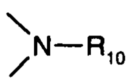

unterbrochenes $C_3$-$C_{18}$-Alkyl; durch Sauerstoff, Schwefel oder

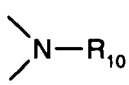

unterbrochenes $C_3$-$C_{18}$-Alkoxy; durch Sauerstoff, Schwefel oder

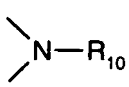

unterbrochenes $C_3$-$C_{18}$-Alkanoyloxy; durch Sauerstoff, Schwefel oder

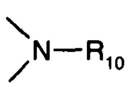

unterbrochenes $C_3$-$C_{18}$-Alkoxycarbonyl; $C_6$-$C_9$-Cycloalkoxycarbonyl, $C_6$-$C_9$-Cycloalkylcarbonyloxy, unsubstituiertes oder durch $C_1$-$C_4$-Alkyl substituiertes Benzoyloxy; $-(CH_2)_p-COR_{11}$ oder $-(CH_2)_qOH$ darstellen, oder ferner die Reste $R_6$ und $R_7$ oder die Reste $R_7$ und $R_8$ oder die Reste $R_8$ und $R_9$ zusammen mit den Kohlenstoffatomen, an die sie gebunden sind, einen Benzoring bilden, $R_3$ zusätzlich einen Rest der Formel II bedeutet, $R_6$ zusätzlich einen Rest der Formel III darstellt,

$R_{10}$ Wasserstoff oder $C_1$-$C_6$-Alkyl ist,

$R_{11}$ Hydroxy,

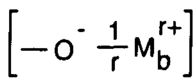 ,

$C_1$-$C_{12}$-Alkoxy,

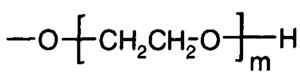 , 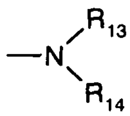

oder einen Rest der Formel IV bedeutet,

$R_{12}$ $-SO_2-$, $-SO_2-R_{16}-SO_2-$,

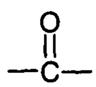 , 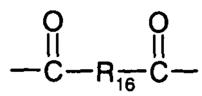 , 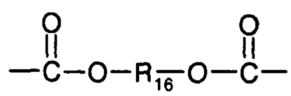

oder

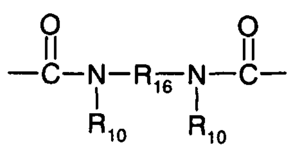

darstellt,

$R_{13}$ und $R_{14}$ unabhängig voneinander Wasserstoff oder $C_1$-$C_8$-Alkyl bedeuten,

$R_{15}$

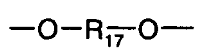

darstellt,

$R_{16}$ $C_2$-$C_{12}$-Alkylen oder $C_5$-$C_{12}$-Cycloalkylen bedeutet,

$R_{17}$ $C_2$-$C_8$-Alkylen, durch Sauerstoff oder Schwefel unterbrochenes $C_4$-$C_{12}$-Alkylen darstellt,

$R_{18}$ Halogen, Nitro,

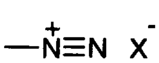 , 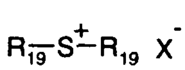 , 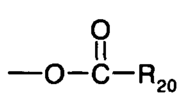 , 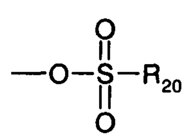

oder $C_1$-$C_{12}$-Alkoxy bedeutet,

$R_{19}$ $C_1$-$C_{18}$-Alkyl, Phenyl oder $C_5$-$C_8$-Cycloalkyl darstellt,
$R_{20}$ $C_1$-$C_{18}$-Alkyl, unsubstituiertes oder durch $C_1$-$C_4$-Alkyl, Fluor, Chlor oder Nitro substituiertes Phenyl; $C_5$-$C_8$-Cycloalkyl, durch Fluor substituiertes $C_1$-$C_{12}$-Alkyl bedeutet,
$R_{21}$, $R_{22}$, $R_{23}$ und $R_{24}$ unabhängig voneinander Wasserstoff oder $C_1$-$C_{12}$-Alkyl darstellen,
$R_{25}$ $C_1$-$C_{12}$-Alkyl bedeutet,
M Lithium, Natrium, Kalium, Calcium

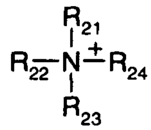

oder $P^+(R_{25})_4$ darstellt,
$M_a$ Natrium oder Kalium ist,
$M_b$ Natrium, Kalium oder Calcium bedeutet,
$X^-$ Chlorid oder Bromid darstellt,
m eine ganze Zahl von 1 bis 15 bedeutet,
n 1 oder 2 ist,
p 0, 1 oder 2 bedeutet,
q 1, 2 oder 3 darstellt, und
r 1 oder 2 ist.

**4.** Verfahren gemäss Anspruch 1, worin
$R_1$ Wasserstoff, Chlor, Carboxy, Nitro, $C_1$-$C_4$-Alkyl, Trifluormethyl oder $C_1$-$C_4$-Alkoxy bedeutet,
$R_2$ Wasserstoff, Chlor, $-SO_3H$, $-SO_3^-M_a^+$, Carboxy, Cyano, Nitro, $C_1$-$C_4$-Alkyl, Trifluormethyl, $C_1$-$C_4$-Alkoxy, Benzyl, Phenyl, Cyclohexyl, $C_1$-$C_4$-Alkylsulfonyl, Phenylsulfonyl, $C_1$-$C_8$-Alkanoyl, $C_1$-$C_8$-Alkoxycarbonyl, $C_1$-$C_8$-Alkanoyloxy, durch Sauerstoff oder Schwefel unterbrochenes $C_3$-$C_8$-Alkyl; durch Sauerstoff oder Schwefel unterbrochenes $C_3$-$C_8$-Alkoxy; durch Sauerstoff oder Schwefel unterbrochenes $C_3$-$C_8$-Alkanoyloxy; durch Sauerstoff oder Schwefel unterbrochenes $C_3$-$C_8$-Alkoxycarbonyl; Cyclohexoxycarbonyl, Cyclohexylcarbonyloxy, Benzoyloxy, darstellt,
$R_3$ Wasserstoff oder einen Rest der Formel II bedeutet,
$R_4$ Wasserstoff, Chlor, Carboxy, Nitro, $C_1$-$C_4$-Alkyl, Trifluormethyl oder $C_1$-$C_4$-Alkoxy darstellt,
$R_6$ Wasserstoff, $C_1$-$C_{12}$-Alkyl, $C_7$-$C_9$-Phenylalkyl, Phenyl, Cyclohexyl, $C_1$-$C_{12}$-Alkoxy, durch Sauerstoff oder Schwefel unterbrochenes $C_3$-$C_{12}$-Alkyl; durch Sauerstoff oder Schwefel unterbrochenes $C_3$-$C_{12}$-Alkoxy; oder einen Rest der Formel III darstellt,
$R_7$ Wasserstoff, Chlor, $C_1$-$C_4$-Alkyl oder $C_1$-$C_4$-Alkoxy bedeutet,
$R_8$ Wasserstoff, $C_1$-$C_{12}$-Alkyl, $C_7$-$C_9$-Phenylalkyl, Phenyl, Cyclohexyl, $C_1$-$C_{12}$-Alkoxy, durch Sauerstoff oder Schwefel unterbrochenes $C_3$-$C_{12}$-Alkyl; durch Sauerstoff oder Schwefel unterbrochenes $C_3$-$C_{12}$-Alkoxy; oder $-(CH_2)_p-COR_{11}$ darstellt,
$R_9$ Wasserstoff, Chlor, $C_1$-$C_4$-Alkyl oder $C_1$-$C_4$-Alkoxy bedeutet,
$R_{11}$ Hydroxy, $C_1$-$C_8$-Alkoxy,

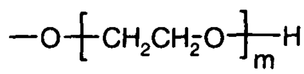

oder einen Rest der Formel IV darstellt,
$R_{12}$ $-SO_2-$,

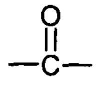 , 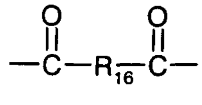 oder 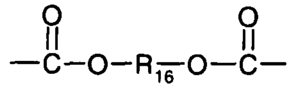

bedeutet,
$R_{15}$

$$-O-R_{17}-O-$$

darstellt,
$R_{16}$ $C_2$-$C_{18}$-Alkylen oder $C_5$-$C_8$-Cycloalkylen bedeutet,
$R_{17}$ $C_2$-$C_8$-Alkylen, durch Sauerstoff unterbrochenes $C_4$-$C_{12}$-Alkylen darstellt,
$R_{18}$ Chlor, Brom, Jod, Nitro,

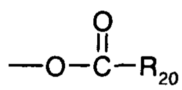 oder 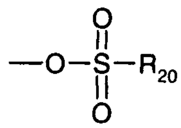

bedeutet,
$R_{20}$ $C_1$-$C_8$-Alkyl, unsubstituiertes oder durch Fluor, Chlor oder Nitro substituiertes Phenyl; oder durch Fluor substituiertes $C_1$-$C_4$-Alkyl darstellt,
M Lithium, Natrium, Kalium oder Calcium bedeutet,
$M_a^+$ Natrium oder Kalium darstellt,
m eine ganze Zahl von 1 bis 15 bedeutet,
n 1 oder 2 ist, und
p 1 oder 2 bedeutet.

**5.** Verfahren gemäss Anspruch 1, worin $R_1$, $R_4$, $R_7$ und $R_9$ Wasserstoff bedeuten.

**6.** Verfahren gemäss Anspruch 1, worin $R_{18}$ Nitro, Chlor oder Brom darstellt.

**7.** Verfahren gemäss Anspruch 1, worin
$R_1$ Wasserstoff oder $C_1$-$C_4$-Alkyl bedeutet,
$R_2$ Wasserstoff, Chlor, $-SO_3H$, $-SO_3^-M_a^+$, Carboxy, Cyano, Nitro, $C_1$-$C_4$-Alkyl, Trifluormethyl, $C_1$-$C_4$-Alkoxy, $C_1$-$C_4$-Alkanoyl, $C_1$-$C_4$-Alkoxycarbonyl, durch Sauerstoff unterbrochenes $C_3$-$C_8$-Alkyl; oder durch Sauerstoff unterbrochenes $C_3$-$C_8$-Alkoxy darstellt,
$R_3$ Wasserstoff bedeutet,
$R_4$ Wasserstoff oder $C_1$-$C_4$-Alkyl darstellt,
$R_6$ Wasserstoff, $C_1$-$C_8$-Alkyl, $C_7$-$C_9$-Phenylalkyl, Phenyl, Cyclohexyl oder einen Rest der Formel III darstellt,
$R_7$ Wasserstoff oder $C_1$-$C_4$-Alkyl bedeutet,
$R_8$ Wasserstoff, $C_1$-$C_{12}$-Alkyl, $C_7$-$C_9$-Phenylalkyl, Phenyl, Cyclohexyl oder $-(CH_2)_p-COR_{11}$ darstellt,
$R_9$ Wasserstoff oder $C_1$-$C_4$-Alkyl bedeutet,
$R_{11}$ Hydroxy, $C_1$-$C_8$-Alkoxy,

$$-O-\left[CH_2CH_2O\right]_m-H$$

oder einen Rest der Formel IV darstellt,
$R_{15}$

$$-O-R_{17}-O-$$

bedeutet,
$R_{17}$ durch Sauerstoff unterbrochenes $C_4$-$C_{12}$-Alkylen darstellt,
$R_{18}$ Chlor, Brom, Nitro,

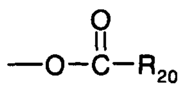 oder 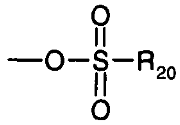

bedeutet,
$R_{20}$ $C_1$-$C_4$-Alkyl, unsubstituiertes oder durch Fluor, Chlor oder Nitro substituiertes Phenyl; oder Trifluormethyl darstellt,
M Lithium, Natrium oder Kalium bedeutet,
$M_a^+$ Natrium oder Kalium darstellt,
m eine ganze Zahl von 1 bis 10 bedeutet,
n 1 ist, und
p 2 bedeutet.

**8.** Verfahren gemäss Anspruch 1, worin
$R_1$ Wasserstoff bedeutet,
$R_2$ Wasserstoff, Chlor, $-SO_3H$, $-SO_3^-$ $M_a^+$, Carboxy, Cyano, Nitro oder Trifluormethyl darstellt,
$R_3$ Wasserstoff bedeutet,
$R_4$ Wasserstoff darstellt,
$R_6$ Wasserstoff, $C_1$-$C_5$-Alkyl, α,α-Dimethylbenzyl oder einen Rest der Formel III darstellt, $R_7$ Wasserstoff ist,
$R_8$ Wasserstoff, $C_1$-$C_8$-Alkyl oder $-(CH_2)_p$-$COR_{11}$ bedeutet,
$R_9$ Wasserstoff ist,
$R_{11}$ $C_1$-$C_8$-Alkoxy,

$$-O-\left[CH_2CH_2O\right]_m-H$$

oder einen Rest der Formel IV darstellt,
$R_{15}$

$$-O-R_{17}-O-$$

bedeutet,
$R_{17}$ durch Sauerstoff unterbrochenes $C_4$-$C_{12}$-Alkylen darstellt,
$R_{18}$ Nitro, Chlor oder Brom bedeutet,
M Lithium oder Natrium ist,
$M_a^+$ Natrium oder Kalium darstellt,
m eine ganze Zahl von 1 bis 10 bedeutet,
n 1 darstellt, und
p 2 ist.

**9.** Verfahren gemäss Anspruch 1, worin die Umsetzung in einem Lösungsmittel erfolgt.

**10.** Verfahren gemäss Anspruch 1, worin das molare Mengenverhältnis der Verbindung der Formel V zu der Azid-Verbindung der Formel IX 1 : 1 bis 1 : 3 beträgt.

**11.** Verfahren gemäss Anspruch 1, worin die Umsetzung in Gegenwart eines Katalysators erfolgt.

## Revendications

**1.** Procédé de préparation d'un composé de formule I

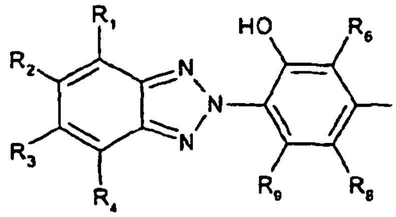 (I)

dans laquelle

$R_1$, $R_2$, $R_3$, $R_4$, $R_6$, $R_7$, $R_8$ et $R_9$ représentent chacun, indépendamment les uns des autres, un atome d'hydrogène, un atome d'halogène, $-SO_3H$, $-SO_3^-M_a^+$, un groupe hydroxy, carboxy, cyano, nitro, alkyle en $C_1$ à $C_{25}$, alkyle en $C_1$ à $C_{25}$ substitué par un atome d'halogène, un groupe hydroxy, carboxy, cyano, alcoxy(en $C_1$ à $C_{18}$) carbonyle, alcoxy en $C_1$ à $C_4$ ou par un groupe amino ; alcényle en $C_2$ à $C_{24}$, phényl(en $C_7$ à $C_9$)alkyle, phényle non substitué ou substitué par un groupe alkyle en $C_1$ à $C_4$, cycloalkyle en $C_5$ à $C_8$ non substitué ou substitué par un groupe alkyle en $C_1$ à $C_4$ ; alcoxy en $C_1$ à $C_{18}$, alkylthio en $C_1$ à $C_{18}$, alkylsulfonyle en $C_1$ à $C_{18}$, phénylsulfonyle non substitué ou substitué par un groupe alkyle en $C_1$ à $C_4$, phénylthio non substitué ou substitué par un groupe alkyle en $C_1$ à $C_4$ ; amino, alkylamino en $C_1$ à $C_4$, di (alkyle en $C_1$ à $C_4$)amino, alcanoyle en $C_1$ à $C_{25}$, alcoxy(en $C_1$ à $C_{25}$)carbonyle, alcanoyloxy en $C_1$ à $C_{25}$, alcanoylamino en $C_1$ à $C_{25}$, alkyle en $C_3$ à $C_{25}$ interrompu par un atome d'oxygène, de soufre ou par

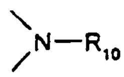 ;

alcoxy en $C_3$ à $C_{25}$ interrompu par un atome d'oxygène, de soufre ou par

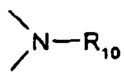 ;

alcanoyloxy en $C_3$ à $C_{25}$ interrompu par un atome d'oxygène, de soufre ou par

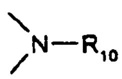 ;

alcoxy(en $C_3$ à $C_{25}$)carbonyle interrompu par un atome d'oxygène, de soufre ou par

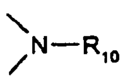 ;

cycloalcoxy(en $C_6$ à $C_9$)carbonyle, cycloalkyl(en $C_6$ à $C_9$)carbonyloxy, benzoyloxy non substitué ou substitué par un groupe alkyle en $C_1$ à $C_{12}$ ; $-(CH_2)_p-COR_{11}$ ou $-(CH_2)_qOH$, ou, en outre, les radicaux $R_6$ et $R_7$ ou les radicaux $R_7$ et $R_8$ ou les radicaux $R_8$ et $R_9$, conjointement avec les atomes de carbone auxquels ils sont liés, forment un noyau benzénique, $R_3$ représente en outre un radical de formule II

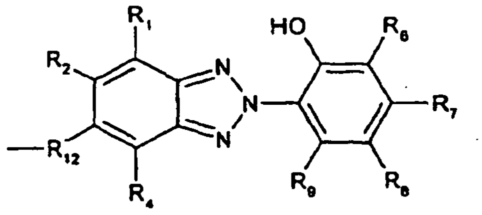

(II),

et $R_6$ représente en outre un radical de formule III

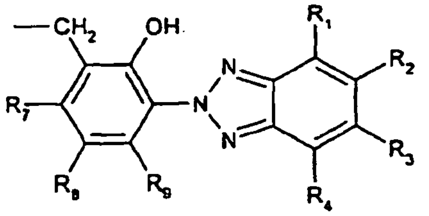

(III),

$R_{10}$ représente un atome d'hydrogène ou un groupe alkyle en $C_1$ à $C_8$,
$R_{11}$ représente un groupe hydroxy,

$$\left[-O^{-}\ \frac{1}{r} M_b^{r+}\right],$$

alcoxy en $C_1$ à $C_{18}$,

$$-O-\left[CH_2CH_2O\right]_m-H\ ,$$

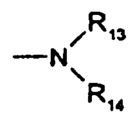

ou un radical de formule IV

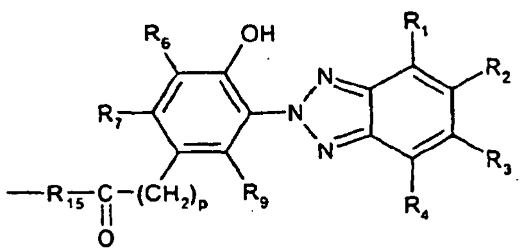

(IV),

$R_{12}$ représente -SO-, $-SO_2-$, $-SO-R_{16}-SO-$, $-SO_2-R_{16}-SO_2-$,

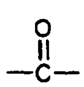 , 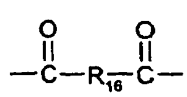 , 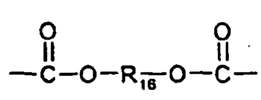 , 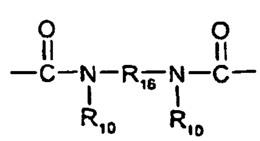 ,

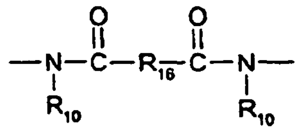

,

$R_{13}$ et $R_{14}$ représentent chacun, indépendamment l'un de l'autre, un atome d'hydrogène ou un groupe alkyle en $C_1$ à $C_{18}$,
$R_{15}$ représente $-O-R_{17}-O-$,
$R_{16}$ représente un groupe alkylène en $C_2$ à $C_{12}$, cycloalkylène en $C_5$ à $C_{12}$, ou un groupe alkylène en $C_8$ à $C_{12}$ interrompu ou terminé par un groupe cyclohexylène ;
$R_{17}$ représente un groupe alkylène en $C_2$ à $C_{12}$, ou alkylène en $C_4$ à $C_{12}$ interrompu par un atome d'oxygène, de soufre ou par

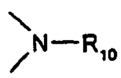

;

$M_a$ représente un cation de métal monovalent,
$M_b$ représente un cation de métal r-valent,
m représente un nombre entier de 1 à 20,
p vaut 0, 1 ou 2,
q vaut 1, 2, 3, 4, 5 ou 6, et
r vaut 1, 2 ou 3,

procédé qui consiste à faire réagir un composé de formule V

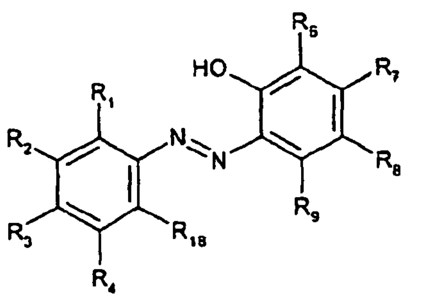

(V)

dans laquelle

$R_1$, $R_2$, $R_3$, $R_4$, $R_6$, $R_7$, $R_8$ et $R_9$ sont tels que définis ci-dessus, $R_3$ représentant en outre un radical de formule VI

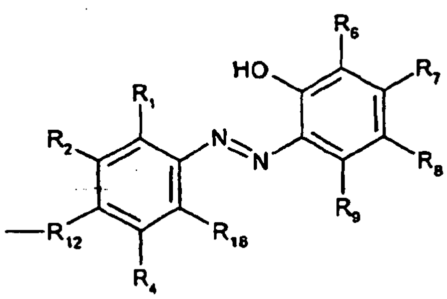

(VI)

$R_6$ représentant en outre un radical de formule VII

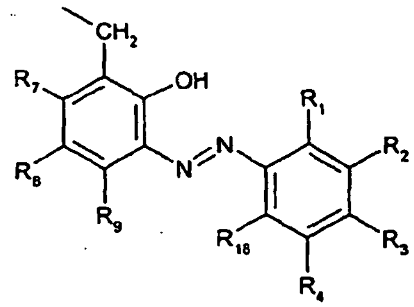

(VII)

et $R_{11}$ représentant en outre un radical de formule VIII

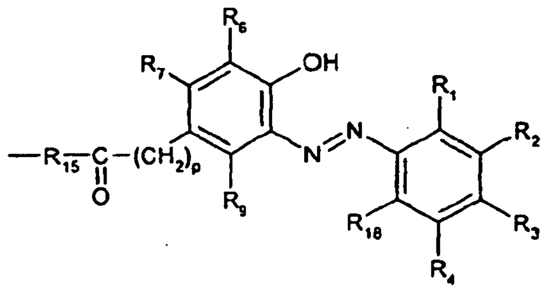

(VIII),

$R_{18}$ représente un atome d'halogène, un groupe nitro,

$-\overset{+}{N}\equiv N\ X^-$, $R_{19}-\overset{+}{S}-R_{19}\ X^-$,

$-SO_3H$,

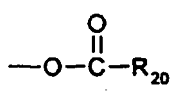
,
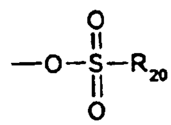

ou un groupe alcoxy en $C_1$ à $C_{18}$,

$R_{19}$ représente un groupe alkyle en $C_1$ à $C_{18}$, phényle non substitué ou substitué par un groupe alkyle en $C_1$ à $C_4$ ; ou cycloalkyle en $C_5$ à $C_8$ non substitué ou substitué par un groupe alkyle en $C_1$ à $C_4$,

$R_{20}$ représente un groupe alkyle en $C_1$ à $C_{18}$, phényle non substitué ou substitué par un groupe alkyle en $C_1$ à $C_4$, halogéno ou nitro ; cycloalkyle en $C_5$ à $C_8$ non substitué ou substitué par un groupe alkyle en $C_1$ à $C_4$ ; ou alkyle en $C_1$ à $C_{18}$ substitué par un atome de fluor, et

X- représente un chlorure, un bromure, un iodure, un hydroxyde, un nitrate ou un nitrite,

avec un composé azide de formule IX

$$M^{n+}\ (N_3^-)_n \qquad \text{(IX)}$$

dans laquelle

M représente un cation de métal n-valent,

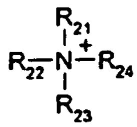

ou $P^+(R_{25})_4$,
$R_{21}$, $R_{22}$, $R_{23}$ et $R_{24}$ représentent chacun, indépendamment les uns des autres, un atome d'hydrogène ou un groupe alkyle en $C_1$ à $C_{18}$,
$R_{25}$ représente un groupe alkyle en $C_1$ à $C_{18}$, et
n vaut 1, 2 ou 3.

**2.** Procédé selon la revendication 1, dans lequel $R_{11}$ représente un groupe hydroxy,

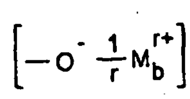,

alcoxy en $C_1$ à $C_{18}$ ou

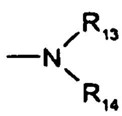

ou un radical de formule IV ;
$R_{13}$ et $R_{14}$ représentent chacun, indépendamment l'un de l'autre, un atome d'hydrogène ou un groupe alkyle en $C_1$ à $C_{18}$,
$M_b$ représente un cation de métal r-valent, et
r vaut 1, 2 ou 3.

**3.** Procédé selon la revendication 1, dans lequel
$R_1$, $R_2$, $R_3$, $R_4$, $R_6$, $R_7$, $R_8$ et $R_9$ représentent chacun, indépendamment les uns des autres, un atome d'hydrogène, un atome d'halogène, $-SO_3H$, $-SO_3$-$Ma^+$, un groupe hydroxy, carboxy, cyano, nitro, alkyle en $C_1$ à $C_{18}$, un groupe alkyle en $C_1$ à $C_{18}$ substitué par un atome de fluor ou un groupe alcoxy en $C_1$ à $C_4$ ; un groupe alcényle en $C_2$ à $C_{18}$, phényl(en $C_7$ à $C_9$)alkyle, phényle non substitué ou substitué par un groupe alkyle en $C_1$ à $C_4$ ; cycloalkyle en $C_5$ à $C_8$, alcoxy en $C_1$ à $C_{18}$, alkylthio en $C_1$ à $C_{18}$, alkylsulfonyle en $C_1$ à $C_{18}$, phénylsulfonyle, phénylthio, alkylamino en $C_1$ à $C_4$, di(alkyle en $C_1$ à $C_4$)amino, alcanoyle en $C_1$ à $C_{18}$, alcoxy(en $C_1$ à $C_{18}$)carbonyle, alcanoyloxy en $C_1$ à $C_{18}$, alcanoylamino en $C_1$ à $C_{18}$, alkyle en $C_3$ à $C_{18}$ interrompu par un atome d'oxygène, de soufre ou par

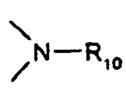 ;

alcoxy en $C_3$ à $C_{18}$ interrompu par un atome d'oxygène, de soufre ou par

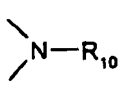 ;

alcanoyloxy en $C_3$ à $C_{18}$ interrompu par un atome d'oxygène, de soufre ou par

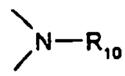 ;

alcoxy(en $C_3$ à $C_{18}$)carbonyle interrompu par un atome d'oxygène, de soufre ou par

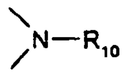 ;

cycloalcoxy(en $C_6$ à $C_9$)carbonyle, cycloalkyl(en $C_6$ à $C_9$)carbonyloxy, benzoyloxy non substitué ou substitué par un groupe alkyle en $C_1$ à $C_4$ ; $-(CH_2)_p-COR_{11}$ ou $-(CH_2)_qOH$, ou, en outre, les radicaux $R_6$ et $R_7$ ou les radicaux $R_7$ et $R_8$ ou les radicaux $R_8$ et $R_9$, conjointement avec les atomes de carbone auxquels ils sont liés, forment un noyau benzénique, $R_3$ représente en outre un radical de formule II et $R_6$ représente en outre un radical de formule III,
$R_{10}$ représente un atome d'hydrogène ou un groupe alkyle en $C_1$ à $C_6$,
$R_{11}$ représente un groupe hydroxy,

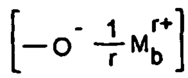 ,

alcoxy en $C_1$ à $C_{12}$,

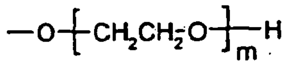 , 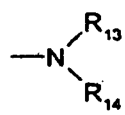

ou un radical de formule IV,
$R_{12}$ représente $-SO_2-$, $-SO_2-R_{16}-SO_2-$,

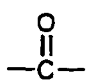 , 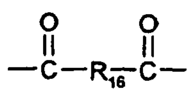 ,

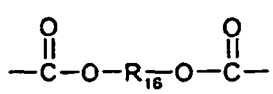 , 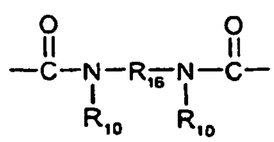 ,

ou
$R_{13}$ et $R_{14}$ représentent chacun, indépendamment l'un de l'autre, un atome d'hydrogène ou un groupe alkyle en $C_1$ à $C_8$, $R_{15}$ représente $-O-R_{17}-O-$,
$R_{16}$ représente un groupe alkylène en $C_2$ à $C_{12}$ ou cycloalkylène en $C_5$ à $C_{12}$,
$R_{17}$ représente un groupe alkylène en $C_2$ à $C_8$, ou alkylène en $C_4$ à $C_{12}$ interrompu par un atome d'oxygène ou de soufre,
$R_{18}$ représente un atome d'halogène, un groupe nitro,

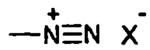 , 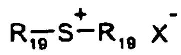 , 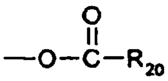 , 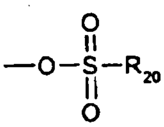

ou un groupe alcoxy en $C_1$ à $C_{12}$,
$R_{19}$ représente un groupe alkyle en $C_1$ à $C_{18}$, phényle ou cycloalkyle en $C_5$ à $C_8$,
$R_{20}$ représente un groupe alkyle en $C_1$ à $C_{18}$, phényle non substitué ou substitué par un groupe alkyle en $C_1$ à $C_4$, un atome de fluor, de chlore ou un groupe nitro ; cycloalkyle en $C_5$ à $C_8$, ou alkyle en $C_1$ à $C_{12}$ substitué par un atome de fluor,
$R_{21}$, $R_{22}$, $R_{23}$ et $R_{24}$ représentent chacun, indépendamment les uns des autres, un atome d'hydrogène ou un groupe alkyle en $C_1$ à $C_{12}$,
$R_{25}$ représente un groupe alkyle en $C_1$ à $C_{12}$,
M représente le lithium, le sodium, le potassium, le calcium,

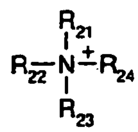

ou $P^+(R_{25})_4$,
$M_a$ représente le sodium ou le potassium,
$M_b$ représente le sodium, le potassium ou le calcium,
$X^-$ représente le chlorure ou le bromure,
m représente un nombre entier de 1 à 15,
n vaut 1 ou 2,
p vaut 0, 1 ou 2,
q vaut 1, 2 ou 3, et
r vaut 1 ou 2.

**4.** Procédé selon la revendication 1, dans lequel
$R_1$ représente un atome d'hydrogène, de chlore, un groupe carboxy, nitro, alkyle en $C_1$ à $C_4$, trifluorométhyle ou alcoxy en $C_1$ à $C_4$,
$R_2$ représente un atome d'hydrogène, de chlore, $-SO_3H$, $-SO_3^-Ma^+$, un groupe carboxy, cyano, nitro, alkyle en $C_1$ à $C_4$, trifluorométhyle, alcoxy en $C_1$ à $C_4$, benzyle, phényle, cyclohexyle, alkylsulfonyle en $C_1$ à $C_4$, phénylsulfonyle, alcanoyle en $C_1$ à $C_8$, alcoxy(en $C_1$ à $C_8$)carbonyle, alcanoyloxy en $C_1$ à $C_8$, alkyle en $C_3$ à $C_8$ interrompu par un atome d'oxygène ou de soufre ; alcoxy en $C_3$ à $C_8$ interrompu par un atome d'oxygène ou de soufre ; alcanoyloxy en $C_3$ à $C_8$ interrompu par un atome d'oxygène ou de soufre ; alcoxy(en $C_3$ à $C_8$)carbonyle interrompu par un atome d'oxygène ou de soufre ; cyclohexyloxycarbonyle, cyclohexylcarbonyloxy ou benzoyloxy,
$R_3$ représente un atome d'hydrogène ou un radical de formule II,
$R_4$ représente un atome d'hydrogène, de chlore, un groupe carboxy, nitro, alkyle en $C_1$ à $C_4$, trifluorométhyle ou alcoxy en $C_1$ à $C_4$,
$R_6$ représente un atome d'hydrogène, un groupe alkyle en $C_1$ à $C_{12}$, phényl(en $C_7$ à $C_9$)alkyle, phényle, cyclohexyle, alcoxy en $C_1$ à $C_{12}$, alkyle en $C_3$ à $C_{12}$ interrompu par un atome d'oxygène ou de soufre ; alcoxy en $C_3$ à $C_{12}$ interrompu par un atome d'oxygène ou de soufre ; ou un radical de formule III,
$R_7$ représente un atome d'hydrogène, de chlore, un groupe alkyle en $C_1$ à $C_4$ ou alcoxy en $C_1$ à $C_4$,
$R_8$ représente un atome d'hydrogène, un groupe alkyle en $C_1$ à $C_{12}$, phényl(en $C_7$ à $C_9$)alkyle, phényle, cyclohexyle, alcoxy en $C_1$ à $C_{12}$, alkyle en $C_3$ à $C_{12}$ interrompu par un atome d'oxygène ou de soufre ; alcoxy en $C_3$ à $C_{12}$ interrompu par un atome d'oxygène ou de soufre ; ou $-(CH_2)_p-COR_{11}$,
$R_9$ représente un atome d'hydrogène, de chlore, un groupe alkyle en $C_1$ à $C_4$ ou alcoxy en $C_1$ à $C_4$,
$R_{11}$ représente un groupe hydroxy, alcoxy en $C_1$ à $C_8$,

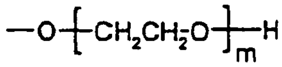

ou un radical de formule IV,
$R_{12}$ représente $-SO_2-$,

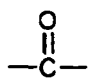 , 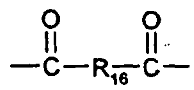 ou 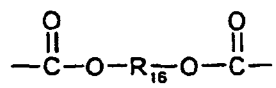 ,

$R_{15}$ représente $-O-R_{17}-O-$,
$R_{16}$ représente un groupe alkylène en $C_2$ à $C_{18}$ ou cycloalkylène en $C_5$ à $C_8$,
$R_{17}$ représente un groupe alkylène en $C_2$ à $C_8$, ou alkylène en $C_4$ à $C_{12}$ interrompu par un atome d'oxygène,
$R_{18}$ représente un atome de chlore, de brome, d'iode, un groupe nitro,

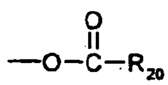 ou 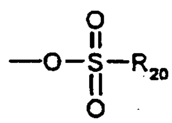 ,

$R_{20}$ représente un groupe alkyle en $C_1$ à $C_8$, phényle non substitué ou substitué par un atome de fluor, de chlore ou un groupe nitro ; ou alkyle en $C_1$ à $C_4$ substitué par un atome de fluor,
M représente le lithium, le sodium, le potassium ou le calcium,
$M_a^+$ représente le sodium ou le potassium,
m représente un nombre entier de 1 à 15,
n vaut 1 ou 2, et
p vaut 1 ou 2.

**5.** Procédé selon la revendication 1, dans lequel $R_1$, $R_4$, $R_7$ et $R_9$ représentent chacun un atome d'hydrogène.

**6.** Procédé selon la revendication 1, dans lequel $R_{18}$ représente un groupe nitro, un atome de chlore ou de brome.

**7.** Procédé selon la revendication 1, dans lequel
$R_1$ représente un atome d'hydrogène ou un groupe alkyle en $C_1$ à $C_4$,
$R_2$ représente un atome d'hydrogène, de chlore, $-SO_3H$, $-SO_3^-Ma^+$, un groupe carboxy, cyano, nitro, alkyle en $C_1$ à $C_4$, trifluorométhyle, alcoxy en $C_1$ à $C_4$, alcanoyle en $C_1$ à $C_4$, alcoxy(en $C_1$ à $C_4$)carbonyle, alkyle en $C_3$ à $C_8$ interrompu par un atome d'oxygène ; ou alcoxy en $C_3$ à $C_8$ interrompu par un atome d'oxygène,
$R_3$ représente un atome d'hydrogène,
$R_4$ représente un atome d'hydrogène ou un groupe alkyle en $C_1$ à $C_4$,
$R_6$ représente un atome d'hydrogène, un groupe alkyle en $C_1$ à $C_8$, phényl(en $C_7$ à $C_9$)alkyle, phényle, cyclohexyle ou un radical de formule III,
$R_7$ représente un atome d'hydrogène ou un groupe alkyle en $C_1$ à $C_4$,
$R_8$ représente un atome d'hydrogène, un groupe alkyle en $C_1$ à $C_{12}$, phényl(en $C_7$ à $C_9$)alkyle, phényle, cyclohexyle ou $-(CH_2)_p-COR_{11}$,
$R_9$ représente un atome d'hydrogène ou un groupe alkyle en $C_1$ à $C_4$,
$R_{11}$ représente un groupe hydroxy, alcoxy en $C_1$ à $C_8$,

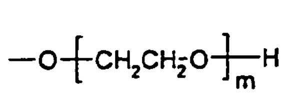

ou un radical de formule IV,
$R_{15}$ représente $-O-R_{17}-O-$,

$R_{17}$ représente un groupe alkylène en $C_4$ à $C_{12}$ interrompu par un atome d'oxygène,
$R_{18}$ représente un atome de chlore, de brome, un groupe nitro,

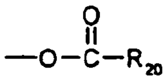 ou 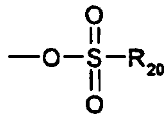 ,

$R_{20}$ représente un groupe alkyle en $C_1$ à $C_4$, phényle non substitué ou substitué par un atome de fluor, de chlore ou un groupe nitro ; ou trifluorométhyle,
M représente le lithium, le sodium ou le potassium,
$M_a^+$ représente le sodium ou le potassium,
m représente un nombre entier de 1 à 10,
n vaut 1, et
p vaut 2.

**8.** Procédé selon la revendication 1, dans lequel
$R_1$ représente un atome d'hydrogène,
$R_2$ représente un atome d'hydrogène, de chlore, $-SO_3H$, $-SO_3^-Ma^+$, un groupe carboxy, cyano, nitro ou trifluorométhyle,
$R_3$ représente un atome d'hydrogène,
$R_4$ représente un atome d'hydrogène,
$R_6$ représente un atome d'hydrogène, un groupe alkyle en $C_1$ à $C_5$, a,a-diméthylbenzyle ou un radical de formule III,
$R_7$ représente un atome d'hydrogène,
$R_8$ représente un atome d'hydrogène, un groupe alkyle en $C_1$ à $C_8$ ou $-(CH_2)_p-COR_{11}$,
$R_9$ représente un atome d'hydrogène,
$R_{11}$ représente un groupe alcoxy en $C_1$ à $C_8$,

$$-O-[CH_2CH_2O]_m-H ,$$

ou un radical de formule IV,
$R_{15}$ représente $-O-R_{17}O-$,
$R_{17}$ représente un groupe alkylène en $C_4$ à $C_{12}$ interrompu par un atome d'oxygène,
$R_{18}$ représente un groupe nitro, un atome de chlore ou de brome,
M représente le lithium ou le sodium,
$M_a^+$ représente le sodium ou le potassium,
m représente un nombre entier de 1 à 10,
n vaut 1, et
p vaut 2.

**9.** Procédé selon la revendication 1, dans lequel la réaction est réalisée dans un solvant.

**10.** Procédé selon la revendication 1, dans lequel le rapport molaire de la quantité de composé de formule V à la quantité du composé azide de formule IX est de 1:1 à 1:3.

**11.** Procédé selon la revendication 1, dans lequel la réaction est réalisée en présence d'un catalyseur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5276161 A
- US 5977219 A
- US 2904544 A


### Non-patent literature cited in the description


- **H. HARTMANN et al.** Journal für praktische Chemie. 1990, vol. 332, 331-335
- **P. SPAGNOLO et al.** *J. Chem. Soc., Perkin Trans,* 1988, vol. I, 2615
- **P. G. HOUGHTON et al.** *J. Chem. Soc., Perkin Trans,* 1985, vol. I, 1471
- **J. H. HALL.** *J. Org. Chem.,* 1986, vol. 33, 2954